(19) **Europäisches Patentamt**
**European Patent Office**
**Office européen des brevets**

(11) **EP 3 146 964 A1**

(12) **EUROPEAN PATENT APPLICATION**
published in accordance with Art. 153(4) EPC

(43) Date of publication:
**29.03.2017 Bulletin 2017/13**

(21) Application number: **15795679.8**

(22) Date of filing: **20.05.2015**

(51) Int Cl.:
*A61K 9/70* (2006.01)     *A61K 47/10* (2017.01)
*A61K 47/14* (2017.01)    *A61K 47/16* (2006.01)
*A61K 47/28* (2006.01)    *A61K 47/32* (2006.01)
*A61L 15/58* (2006.01)    *A61P 17/16* (2006.01)

(86) International application number:
**PCT/JP2015/064544**

(87) International publication number:
**WO 2015/178438 (26.11.2015 Gazette 2015/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **20.05.2014 JP 2014104496**

(71) Applicant: **Alcare Co., Ltd.**
**Sumida-ku**
**Tokyo 131-0046 (JP)**

(72) Inventors:
• **WATANABE, Ryota**
**Tokyo 131-0046 (JP)**
• **OKUYAMA, Wataru**
**Tokyo 131-0046 (JP)**

(74) Representative: **D Young & Co LLP**
**120 Holborn**
**London EC1N 2DY (GB)**

(54) **ADHESIVE AGENT COMPOSITION FOR SKIN AND PATCH MATERIAL FOR SKIN**

(57)     The present invention provides a dermatological adhesive composition having an enhanced skin protective effect. The present invention provides a dermatological adhesive composition comprising a resin component for forming an adhesive and a lamellar structure formed of a lipid composition; a dermatological adhesive composition comprising a resin component for forming an adhesive, and a lipid composition containing at least one selected from the group consisting of sphingolipids and sterols and at least one selected from the group consisting of higher alcohols and surfactants; and a dermatological patch including a base sheet, and an adhesive layer formed of the dermatological adhesive composition and disposed on the base sheet.

**Description**

Technical Field

The present invention relates to dermatological adhesive compositions and dermatological patches. The present invention more specifically relates to a dermatological adhesive composition comprising a lipid composition, and a dermatological patch comprising the dermatological adhesive composition.

Background Art

Patches have been used to fix medical materials and appliances, such as gauze dressings, bandages, plasters, catheters, and ostomy appliances, to skins, and to protect and treat skins, wounds, and portions around stomata.
A typical dermatological adhesive used in the patches includes a resin component, and components giving desired functions to the adhesive.

For example, PTL 1 discloses an invention relating to an adhesive composition for dermatological application comprising predetermined amounts of an elastomer, a moisture absorbing compound, ceramide, and an emulsifier.
For example, PTL 2 discloses an invention relating to a dermatological adhesive comprising 2 to 35 wt% thermoplastic elastomer, 25 to 60 wt% hydrophilic high-molecular compound, 16 to 40 wt% softening agent, and 0.01 to 4.8 wt% physiological activator, such as ceramide.

Citation List

Patent Literature

PTL 1: Japanese Patent Application Laid-Open No. 2006-263042
PTL 2: Re-publication of PCT International Publication No. 2009/087877

Summary of Invention

The present invention provides a dermatological adhesive composition comprising a resin component and a lamellar structure comprising a lipid composition.
The present invention also provides a dermatological adhesive composition comprising a resin component, and a lipid composition comprising at least one lipid selected from the group consisting of sphingolipids and sterols and at least one selected from higher alcohols and surfactants.
Furthermore, the present invention provides a dermatological patch including a base sheet, and an adhesive layer formed of the dermatological adhesive composition and disposed on the base sheet.

Advantageous Effects of Invention

The present invention can provide a dermatological adhesive composition having an enhanced skin protective effect.

Brief Description of Drawings

[Fig. 1] Fig. 1 is an image taken through examination of the adhesive layer in Example 11 with a polarizing microscope.
[Fig. 2] Fig. 2 is an image taken through examination of the adhesive layer in Example 22 with a polarizing microscope.
[Fig. 3] Fig. 3 is an image taken through examination of the adhesive layer in Example 24 with a polarizing microscope.
[Fig. 4] Fig. 4 is an image taken through examination of the adhesive layer in Example 25 with a polarizing microscope.
[Fig. 5] Fig. 5 is an image taken through examination of the adhesive layer in Example 35 with a polarizing microscope.

Description of Embodiments

Preferred embodiments for implementing the present invention will now be described. The embodiments described below are exemplary embodiments of the present invention, and should not be construed to limit the scope of the present invention.

A lamellar structure contained in the dermatological adhesive composition according to the embodiment for implementing the present invention may comprise a lipid composition comprising a sphingolipid and/or a sterol. The lamellar structure may comprise a lipid composition comprising at least one selected from the group consisting of sphingolipids, sterols, and surfactants; and a higher alcohol.

The lamellar structure may comprise a lipid composition comprising a glycerol fatty acid ester, and an acylated lactic acid and/or a salt thereof.

The lamellar structure may comprise a lipid composition comprising polyoxyalkylene alkyl ether or polyoxyalkylene glycol fatty acid ester as a surfactant.

The lamellar structure may comprise a lipid composition comprising at least one selected from the group consisting of sphingolipids, sterols, and surfactants in a total amount of 5 to 80 mass%, and higher alcohols in a total amount of 10 to 50 mass%.

The lamellar structure may be formed of a lipid composition comprising at least one selected from sphingolipids, sterols, and glycerol fatty acid esters in a total amount of 5 to 80 mass%, and higher alcohols in a total amount of 10 to 50 mass%.

The resin component contained in the dermatological adhesive composition according to the embodiment for implementing the present invention may be an acrylic resin serving as a resin component for forming a hot-melt adhesive, or may be a thermoplastic styrene-based elastomer serving as a resin component for forming a hot-melt adhesive.

The dermatological adhesive composition according to the embodiment for implementing the present invention may further contain a hydrophilic polymer.

The dermatological adhesive composition according to the embodiment for implementing the present invention may comprise a resin component, and a lipid composition comprising at least one lipid selected from the group consisting of sphingolipids and sterols and at least one selected from the group consisting of higher alcohols and surfactants. In this case, the surfactant may be at least one surfactant selected from the group consisting of glycerol fatty acid esters, acylated lactic acids and/or salts thereof, polyoxyalkylene alkyl ethers, and polyoxyalkylene glycol fatty acid esters.

The dermatological adhesive is applied to the skin, and is removed from the skin after use.

The dermatological adhesive is used, for example, to fix patches of ostomy appliances or wound dressings to the skin. Because the patches are fixed to the skin having moisture, such as sweat, and oil content, such as sebum, and the dermatological adhesive should have a predetermined adhesiveness.

Such use of the dermatological adhesive may cause loss of skin protective components in peeling the dermatological adhesive from the skin, thereby reducing the skin barrier function.

Compounding of sphingolipid, such as ceramide, in conventional dermatological adhesive compositions has been examined to maintain the skin barrier function. The patch comprising dermatological adhesives containing sphingolipid, such as ceramide, seems to maintain the skin barrier function due to the sphingolipid, while being applied to the skin. However, skin protective components are removed from the skin due to the adhesiveness of the adhesive when the patch is removed after use. This may eventually damage the skin barrier function. The adhesive has such inherent problems.

In consideration of such circumstances, the dermatological adhesive composition according to the embodiment of the present invention is mainly directed to provide a dermatological adhesive composition having an enhanced skin protective effect. Surprisingly, the present inventor, who has conducted extensive research in consideration of the circumstances above, has successfully prepared a dermatological adhesive composition in which a lamella structure, which intercorneocyte lipid forms, or a lipid composition having a specific component is included. The present inventor has found that use of the dermatological adhesive composition enhances the recovery rate of transepidermal water loss (hereinafter, abbreviated to TEWL), and has completed the dermatological adhesive composition according to the present embodiment. TEWL will be described in detail later.

<Dermatological adhesive composition>

The dermatological adhesive composition according to the first aspect of the present embodiment comprises a resin component, and a lamellar structure comprises a lipid composition. The lamellar structure comprises a lipid composition may exist in the resin component, or the adhesive may form lamella structure as a whole..

The lamellar structure preferably comprises a lipid composition comprising a sphingolipid and/or a sterol. The lamellar structure also preferably comprises a lipid composition comprising at least one selected from sphingolipids, sterols, and surfactants; and higher alcohol. Furthermore, the lamellar structure also preferably comprises a lipid composition comprising a glycerol fatty acid ester, and an acylated lactic acid and/or a salt thereof. Furthermore, the lamellar structure also preferably comprises a lipid composition comprising polyoxyalkylene alkyl ether or polyoxyalkylene glycol fatty acid ester as a surfactant.

The dermatological adhesive composition according to the second aspect of the present embodiment comprises a resin component; and a lipid composition comprising at least one selected from the group consisting of sphingolipids and sterols and at least one selected from the group consisting of higher alcohols and surfactants. In the dermatological adhesive composition according to the second aspect, the lipid composition contained in the dermatological adhesive composition is preferably configured to form a lamella structure in the resin component.

The dermatological adhesive composition of the present embodiment (hereinafter, referred to as "adhesive composition" in some cases) comprises a lipid composition comprising a lamellar structure or a specific component, and thus can have a skin protective effect more significantly enhanced.

The stratum corneum in the epidermis of the skin is formed of corneocytes, and intercorneocyte lipid exists in corneocytes. The intercorneocyte lipid has a lamella structure , in which moisture content and the oil content (lipid) alternatingly stack in layers.

When a dermatological adhesive comprising this adhesive composition (hereinafter, simply referred to as "adhesive" in some cases) is applied to the skin, it has affinity with the skin thereby showing an enhanced skin protective effect. This seems to be because the adhesive composition of the present embodiment contains a lamella structure comprising a lipid composition that has a structure similar or close to the structure of the intercorneocyte lipid or a specific lipid composition capable of forming a lamellar structure similar or close to the structure of the intercorneocyte lipid..

It is also believed that when the adhesive comprising the adhesive composition of the present embodiment is applied to the skin, the lamellar structure formed of the lipid composition or the specific lipid composition in the adhesive composition penetrates into the skin or remains on the surface of the skin. It is believed that the lamellar structure or the specific lipid composition can impart a skin moisturizing effect and a skin barrier effect to the resulting adhesive.

Adhesive compositions containing sphingolipid, such as ceramide, that can impart a skin moisturizing effect and a skin barrier effect to the resulting adhesive have been examined. The present inventors, who has conducted extensive research to attain an adhesive composition having an enhanced skin protective effect, has found that a lamella structure is barely formed if sphingolipid, such as ceramide, alone is compounded, because the formation of the lamella structure is affected by other components contained in the adhesive composition or the process in formation of the adhesive from the adhesive composition.

For these reasons, the dermatological adhesive composition according to the present embodiment is implemented as an adhesive composition comprising a resin component and a lamellar structure comprising a lipid composition, or an adhesive composition comprising the resin component and a lipid composition capable of forming a lamella structure.

It is further confirmed that the skin barrier function is well recovered after the adhesive comprising the adhesive composition of the present embodiment is applied to the skin and is removed from the skin. It is not always the case that the recovery rate of the skin barrier function is enhanced in the presence of the same lamella structure as that of intercorneocyte lipid in the adhesive. Therefore the recovery rate of the skin barrier function can be enhanced by an interaction between the resin component and a lamellar structure comprising a lipid composition or a lipid composition capable of forming such a lamellar structure.

The stratum corneum of the skin has a barrier function to prevent invasion of foreign substances to the skin and evaporation of the moisture content needed for the human body. In particular, the TEWL is highly correlated with the barrier function of the stratum corneum as an index for evaluating such a skin barrier function, and the effectiveness of the TEWL is broadly recognized among medical professionals and skin care instructors or practioners, such as beauty counselors. A higher TEWL recovery rate indicates enhanced recovery of the skin barrier function.

As described above, since the dermatological adhesive is removed from the skin after use, the adhesive may damage the skin. The dermatological adhesive is used, for example, to fix ostomy appliances or patches of wound dressings to the skin. Because the patches are fixed to the skin having a moisture content, such as sweat, and an oil content, such as sebum, the dermatological adhesive should have a certain range of adhesiveness. For this reason, much more concerns are rising that peel-off of the adhesive from the skin may damage the skin. The dermatological adhesive has such inherent problems.

A dermatological adhesive having an adhesiveness to the skin of less than about 0.5 N/25 mm generally alleviates damage to the skin occurring in peeling the adhesive and the TEWL will recover relatively quickly. However, the adhesive having such a low adhesiveness readily peels off and is not always suitable for practical use. A dermatological adhesive having an adhesiveness to the skin of 0.5 N/25 mm or more, for example, will remove protective components from the skin when it is removed. In such a case, the TEWL barely recovers. For this reason, the dermatological adhesive composition of the present embodiment preferably comprises an adhesive composition having an adhesiveness to the skin of preferably 0.5 N/25 mm or more, more preferably 1 N/25 mm or more, still more preferably 2 N/25 mm or more.

The resin component of the present embodiment is not limited as described later in Examples. For this reason, preferred examples will now be described in detail from the viewpoint of the lamella structure forming ability of the lipid composition forming a lamellar structure contained in the dermatological adhesive composition of the first aspect and the lipid composition contained in the dermatological adhesive composition of the second aspect.

[Lipid composition]

Preferred examples of the lipid composition used in the adhesive composition of the present embodiment include lipid compositions containing sphingolipids and/or sterols; lipid compositions containing at least one selected from sphingolipids, sterols, and surfactants, and lipid compositions containing higher alcohols; and lipid compositions containing glycerol fatty acid esters and acylated lactic acids and/or salts thereof. Also preferred are lipid compositions containing polyoxyalkylene alkyl ethers or polyoxyalkylene glycol fatty acid esters as surfactants.

More preferred is a lipid composition comprising at least one selected from ceramides, cholesterols, and polyglycerol fatty acid esters; and higher alcohols. Still more preferred is a lipid composition comprising at least one selected from ceramides, cholesterols, and polyglycerol fatty acid esters; a higher alcohol; and acylated lactic acids and/or salts thereof.

Still more preferred is a lipid composition comprising a ceramide and/or cholesterol; a higher alcohol; a polyglycerol fatty acid ester; and an acylated lactic acid and/or a salt thereof.

In a lipid composition comprising both the sphingolipid and sterol, the total content of sphingolipid and sterol is preferably 10 to 80 mass%, more preferably 20 to 80 mass%, still more preferably 30 to 70 mass%. In a lipid composition comprising either sphingolipid or sterol, the content of sphingolipid or sterol is 1 to 50 mass%, more preferably 1 to 30 mass%, still more preferably 2 to 30 mass%, further more preferably 5 to 20 mass%. Such range of the total content is true of the case where Ceramide as a preferred sphingolipid and cholesterol as a preferred sterol can is used.

In a lipid composition containing a higher alcohol, the content of the higher alcohol in the lipid composition is preferably 10 to 60 mass%, more preferably 20 to 50 mass%, still more preferably 30 to 50 mass%.

In a lipid composition containing a surfactant, the content of the surfactant in the lipid composition is preferably 10 to 60 mass%, more preferably 20 to 50 mass%, still more preferably 30 to 50 mass%.

In a lipid composition containing a glycerol fatty acid ester as a surfactant, the content of the glycerol fatty acid ester in the lipid composition is preferably 10 to 50 mass%, more preferably 20 to 40 mass%, still more preferably 30 to 40 mass%.

In a lipid composition containing an acylated lactic acid and/or a salt thereof as a surfactant, the total content of the acylated lactic acid and/or a salt thereof in the lipid composition is preferably 1 to 30 mass%, more preferably 2 to 30 mass%, still more preferably 5 to 20 mass%.

Also preferred is a lipid composition containing at least one selected from the group consisting of sphingolipids, sterols, and surfactants in a total amount of 5 to 80 mass%, and higher alcohols in a total amount of 10 to 50 mass%.

Also preferred is a lipid composition containing at least one selected from the group consisting of sphingolipids, sterols, and glycerol fatty acid esters in a total amount of 5 to 80 mass%, and higher alcohols in a total amount of 10 to 50 mass%.

In a lipid composition containing a sphingolipid and/or a sterol; a higher alcohol; and a surfactant, a preferred mass ratio is [sphingolipid and/or sterol]:[higher alcohol]:[surfactant]=1 to 5:2 to 5:3 to 5.

In a lipid composition containing a sphingolipid and/or a sterol; a higher alcohol; a glycerol fatty acid ester; and an acylated lactic acid and/or a salt thereof, a preferred mass ratio is [sphingolipid and/or sterol]:[higher alcohol]:[glycerol fatty acid ester]:[acylated lactic acid and/or salt thereof]=1:4 to 5:3 to 4:1 to 2.

In a lipid composition containing a higher alcohol, a glycerol fatty acid ester, and an acylated lactic acid and/or a salt thereof, the proportions of these components preferably decrease in this order; a preferred mass ratio is [higher alcohol]: [glycerol fatty acid ester]: [acylated lactic acid and/or salt thereof]=4 to 5:3 to 4:1 to 2.

(Sphingolipid and/or sterol)

The sphingolipids and sterols usable in the lipid composition are categorized into intercorneocyte lipids or analogs thereof. For this reason, sphingolipids and sterols are components suitable for a lipid composition that can form a lamella structure. Ceramide, i.e., one of the sphingolipids will enhance the skin barrier function, and have a noticeable effect in replenishing the intercorneocyte lipid.

The sphingolipids preferably used in the present embodiment are a ceramide formed of a sphingoid base and a fatty acid bonded thereto through an amido bond, and a sphingoglycolipid formed of a ceramide and sugar bonded thereto. The ceramide refers to a group of compounds each formed of a fatty acid bonded to an amino group of the sphingoid base through an amido bond, such as sphingosine (2-amino-4-octadecen-1,3-diol), dihydrosphingosine (2-amino-4-octadecan-1,3-diol), or phytosphingosine. Natural ceramides, pseudo-ceramides, and mixtures thereof can be used.

Since ceramides contribute to the skin moisturizing function and the skin barrier function, ceramides are preferably contained in the adhesive composition according to the present embodiment. The sphingoid bases of the ceramides also seem to have a skin protective effect.

Ceramides used in the present embodiment are of types 1 to 6 having hydrocarbon groups with different structures derived from fatty acids bonded to the amino groups of the sphingoid bases, such as sphingosine and sphinganine, (hereinafter, referred to as "Ceramides 1 to 6" in some cases). These ceramides can be used alone or in combination in the present embodiment.

More preferred in the adhesive composition according to the present embodiment is use of Ceramide 2 because of its noticeable effect of replenishing the intercorneocyte lipid.

Examples of the sterol used in the present embodiment include cholesterol, dihydrocholesterol, lanosterol, dihydrolanosterol, desmosterol, stigmasterol, sitosterol, campesterol, brassicasterol, and ergosterol. One or more of these sterols can be used. Among these sterols, preferred is cholesterol.

(Surfactant)

The lipid composition of the adhesive composition according to the present embodiment preferably contains a surfactant to form a lamellar structure. Any surfactant that can demonstrate the advantageous effects of the adhesive composition according to the present embodiment can be used. Ionic surfactants (anionic surfactants, cationic surfactants, and amphoteric surfactants) and nonionic surfactants can be appropriately used. More preferred is use of nonionic surfactants or anionic surfactants, and still more preferred is use of nonionic surfactants.

(Anionic surfactant)

Examples of the anionic surfactants include higher alkyl sulfate ester salts, such as sodium lauryl sulfate and potassium lauryl sulfate; alkylether sulfate ester salts, such as POE lauryl sulfurate triethanolamine and POE sodium lauryl sulfate; alkylbenzene sulfonates, such as linear sodium dodecylbenzenesulfonate, linear triethanolamine dodecylbenzenesulfonate, and linear dodecylbenzene sulfonate; and acylated lactate, such as sodium lauroyllactate, sodium stearoyllactate, polyoxyethylene sorbitan fatty acid esters, Polysorbate-60, and Polysorbate-80. Among these anionic surfactants, preferred is use of acylated lactic acids and/or acyllactate in the adhesive composition according to the present embodiment. POE is an abbreviation of polyoxyethylene, which will also be used below.

(Acylated lactic acids and/or acyllactate)

The lipid composition preferably contains acylated lactic acids and/or acyllactate, that is, one or both of acylated lactic acids and acyllactate.

Acylated lactic acids and/or salts thereof contained in the lipid composition can generate an emulsion of a lipid composition, which facilitates formation of a lamella structure. Preferred from such a viewpoint is use of acylated lactic acids and/or acyllactate in combination with polyglycerol fatty acid ester.

The carbon chain of acyl group in acylated lactic acids and/or acyllactate may be linear, branched, or cyclic. Specific examples thereof include acetyl, propanoyl, butyryl, pivaloyl, valeryl, hexanoyl, stearoyl, isostearoyl, capryolyl, caprinoyl, lauroyl, myristoyl, palmitoyl, behenoyl, isopalmitoyl, linoleoyl, and oleoyl groups.

Examples of suitable acyl groups include stearoyl, isostearoyl, capryolyl, caprinoyl, lauroyl, myristoyl, palmitoyl, behenoyl, isopalmitoyl, linoleoyl, and oleoyl groups. Among these acyl groups, more preferred are a stearoyl group and an isostearoyl group.

Specific examples of preferred acylated lactic acids include stearoyllactic acid, isostearoyllactic acid, capryolyllactic acid, behenoyllactic acid, cocoyllactic acid, lauroyllactic acid, oleoyllactic acid, 2-ethylhexanoyllactic acid, myristoyllactic acid, palmitoyllactic acid, 12-hydroxystearoyllactic acid, and linoleoyllactic acid. Among these acylated lactic acids, more preferred are stearoyllactic acid and isostearoyllactic acid.

Examples of suitable salts of acylated lactate include alkali metal salts, such as sodium salts and potassium salts; alkaline earth metal salts, such as calcium salts and magnesium salts; organic amine salts, such as ammonium salts, triethylamine salts, and triethanolamine salts; and basic amino acid salts, such as lysine and arginine. Among these salts, preferred are alkali metal salts, and more preferred are sodium salts.

These acylated lactic acids and acylated lactate salts can be used alone or in combination. Among these acylated lactic acids and acylated lactate salts, still more preferred is use of sodium stearoyl lactate.

(Cationic surfactant)

Examples of the cationic surfactant include alkyltrimethylammonium salts, such as stearyltrimethylammonium chloride and lauryltrimethylammonium chloride; quaternary ammonium salts, such as alkyldimethylbenzylammonium; and alkylamine salts.

(Amphoteric surfactant)

Examples of the amphoteric surfactant include betaine surfactants, such as 2-heptadecyl-N-carboxymethyl-N-hydroxyethylimidazoliniumbetaine, betaine lauryldimethylaminoacetate, alkylbetaine, amidebetaine, and sulfobetaine.

(Nonionic surfactant)

Examples of the nonionic surfactant include glycerol fatty acid esters and alkylene glycol adducts thereof, polyglycerol fatty acid esters and alkylene glycol adducts thereof, propylene glycol fatty acid esters and alkylene glycol adducts thereof, pentaerythritol fatty acid esters (such as pentaerythrityl tetraisostearate), sorbitan fatty acid esters and alkylene glycol adducts thereof, fatty acid esters of sorbitol and alkylene glycol adducts thereof (such as sorbitan fatty acid esters, such as sorbitan monooleate, sorbitan monostearate, sorbitan monoisostearate, sorbitan monopalmitate, and sorbitan monolaurate), polyalkylene glycol fatty acid esters, polyoxyalkylene alkyl ether, glycerol alkyl ether, polyoxyethylene alkylphenyl ether, polyoxyalkylene-modified silicone, polyoxyalkylene/alkyl co-modified silicone, glycerol-modified silicone, and polyoxyalkylene/silicone co-modified silicone. Particularly preferred is use of glycerol fatty acid esters, sorbitan fatty acid esters and alkylene glycol adducts thereof, polyoxyalkylene alkyl ether, or polyalkylene glycol fatty acid esters.
The preferable hydrophile-lipophile balance (HLB) value of nonionic surfactant is, though it is not limited to, 1 to 18, more preferably 2 to 15, still more preferably 2 to 10, still more preferably 2 to 8.
The HLB value used in the present embodiment can be any known nominal value, for example, calculated by an emulsion method (see "Handobukku -Keshohin/Seizai Zairyo- (Handbook of Raw Materials for Cosmetics and Drug Products)", Nikko Chemicals Co., Ltd. (revision published in February 1, 1977)). In the measurement of the HLB value, sorbitan monostearate (such as NIKKOL SS-10 made by Nikko Chemicals Co., Ltd., HLB value: 4.7) and polyoxyethylenesorbitan monostearate (such as NIKKOL TS-10 made by Nikko Chemicals Co., Ltd., HLB value: 14.9) are used in combination as standard surfactants. Liquid paraffin is used as an object to be emulsified. If liquid paraffin may have different HLB values for every type or lot, the HLB value is measured for each type or lot. Liquid paraffin is emulsified with these two standard surfactants, and the optimal proportion of these surfactants is determined. The HLB value needed for liquid paraffin (HLB value of the object to be emulsified) is then determined. The value is calculated from Expression (1):

[Expression 1]

$$\text{HLB value of liquid paraffin} = \frac{(\text{HLB value of polyoxyethylenesorbitan monostearate}) \times (\text{amount used (mass\%)}) + (\text{sorbitan monostearate}) \times (\text{amount used (mass\%)})}{100}$$

The HLB value needed for liquid paraffin is usually about 10.1 to 10.3 although it varies according to the type and lot. The HLB value of the surfactant is measured with a liquid paraffin having a known HLB value. The liquid paraffin is emulsified with a combination of a surfactant and sorbitan monostearate in case the surfactant is hydrophilic or a combination of a surfactant and polyoxyethylenesorbitan monostearate in case the surfactant is hydrophilic to determine optimal proportions of the surfactants to yield stable emulsion. The HLB value of the surfactant is calculated according to Expression 1.

(Glycerol fatty acid ester)

The lipid composition preferably contains glycerol fatty acid esters, which are esters of glycerol or polyglycerol having a degree of polymerization of 2 or more and fatty acids, among the nonionic surfactants. Examples of the glycerol fatty acid esters include monoglycerol fatty acid esters and polyglycerol fatty acid esters.
The glycerol fatty acid ester may be formed of two or more fatty acid moieties. Examples of the fatty acid include acetic acid, propionic acid, butyric acid, stearic acid, isostearic acid, lauric acid, myristic acid, oleic acid, caprylic acid, and capric acid. Among these fatty acids, preferred is stearic acid.
Examples of the monoglycerol fatty acid esters include glyceryl stearate, glyceryl isostearate, glyceryl laurate, glyceryl myristate, glyceryl oleate, glyceryl caprylate, and glyceryl caproate.
Examples of the polyglycerol fatty acid esters include polyglyceryl stearates, polyglyceryl isostearates, polyglyceryl laurates, polyglyceryl myristates, polyglyceryl oleates, polyglyceryl caprylates, and polyglyceryl caproates.
These glycerol fatty acid esters can be used alone or in combination. Among these glycerol fatty acid esters, preferred are polyglycerol fatty acid esters, and more preferred are polyglyceryl stearates.
Examples of preferred polyglyceryl stearates include polyglyceryl-2, -4, -5, -6, and -10 monostearates, polyglyceryl-2, -3, -6, -9, and -10 distearates, polyglyceryl-4, -6, and -10 tristearates, and polyglyceryl-4, -6, and -10 pentastearates.

Examples of preferred polyglyceryl isostearates include polyglyceryl-2, -4, -6, and -10 monoisostearates, polyglyceryl-2, -3, -6, and -10 diisostearates, polyglyceryl-2 and -10 triisostearates, and polyglyceryl-2 tetraisostearate.

Examples of suitable polyglyceryl laurates include polyglyceryl-2, -3, -4, -5, -6, and -10 monolaurates, and polyglyceryl-5 and -10 dilaurates.

Examples of preferred polyglyceryl myristates include polyglyceryl-5, -6, and -10 monomyristates, polyglyceryl-10 dimyristate, and polyglyceryl-5 trimyristate.

The "n" of "polyglyceryl-n", such as polyglyceryl-2, -3, -4, as compound names of the specific examples of the polyglycerol fatty acid esters indicates the average number of glycerols polymerized (average degree of polymerization). For example, polyglyceryl-10 pentastearate refers to an ester compound (decaglycerol pentastearate) of a decamer of glycerol (decaglycerol) and five molecules of stearic acid.

These polyglycerol fatty acid esters can be used alone or in combination.

Among these polyglycerol fatty acid esters used in the adhesive composition according to the present embodiment, more preferred are decaglycerol fatty acid esters and polyglyceryl-4, -6, and -10 pentastearates, and still more preferred are polyglyceryl-10 pentastearate (decaglycerol pentastearate).

(Polyoxyalkylene alkyl ether)

The polyoxyalkylene alkyl ether used in the present embodiment is prepared through addition polymerization of alkylene oxide and higher alcohol.

The polyoxyalkylene alkyl ether may have a linear or branched alkyl group. Specific examples of the alkyl group include octyl, nonyl, decyl, undecyl, dodecyl (lauryl), tridecyl, tetradecyl (myristyl), pentadecyl, hexadecyl (cetyl, palmityl), heptadecyl, octadecyl (stearyl), nonadecyl, icosyl, and behenyl groups (these alkyl groups include linear and branched groups thereof).

The polyoxyalkylene alkyl ether may be formed of a linear or branched moiety of alkylene oxide. Specific examples thereof include ethylene oxide, propylene oxide, butylene oxide, and $-CH(CH_3)CH_2O-$.

The polyoxyalkylene alkyl ether used in the adhesive composition according to the present embodiment may include a copolymer of a higher alcohol and one alkylene oxide, or may include a random copolymer of a higher alcohol and two or more alkylene oxides.

Examples of suitable polyoxyalkylene alkyl ether used in the present embodiment include POE(2) lauryl ether, POE(9) lauryl ether, POE(21) lauryl ether, POE(10) cetyl ether, POE(20) cetyl ether, POE(30) cetyl ether, POE(4) stearyl ether, and POE(20) stearyl ether. Particularly preferred is use of POE(9) lauryl ether and POE(4) stearyl ether.

(Polyalkylene glycol fatty acid ester)

Known polyalkylene glycol fatty acid esters can be used in the present embodiment as long as they do not inhibit the advantageous effects of the invention. The polyalkylene glycol fatty acid ester refers to an ester of a saturated or unsaturated fatty acid and a polyalkylene glycol. The polyalkylene glycol fatty acid ester has a polyalkylene group having a repetition number of preferably 8 to 100, more preferably 10 to 80. Examples of the polyalkylene glycol fatty acid ester can include polyethylene glycol fatty acid esters, such as polyethylene glycol monolaurate, polyethylene glycol monostearate, and polyethylene glycol monooleate; and propylene glycol fatty acid esters, such as propylene glycol monostearate, propylene glycol monolaurate, and propylene glycol monooleate. Among these, preferred is use of polyethylene glycol (45 E.O.) monostearate.

(Higher alcohol)

The lipid composition preferably contains a higher alcohol. Throughout the specification, the higher alcohol refers to an alcohol having 6 or more carbon atoms.

Any higher alcohol can be used. Aliphatic alcohols having 6 to 40 carbon atoms are preferred, those having 8 to 36 carbon atoms are more preferred, and those having 10 to 24 carbon atoms are still more preferred. The aliphatic alcohol may have a linear structure, a branched structure, or a cyclic structure. The aliphatic alcohol may be an unsaturated aliphatic alcohol having one or more double bonds or triple bonds, or may be a saturated aliphatic alcohol.

Specific examples of preferred higher alcohols include capryl alcohol, lauryl alcohol, myristyl alcohol, cetyl alcohol (cetanol), stearyl alcohol, oleyl alcohol, behenyl alcohol (1-docosanol), arachyl alcohol, 2-octyllauryl alcohol, 2-hexyldecyl alcohol, isostearyl alcohol, and octyldodecanol. These higher alcohols can be used alone or in combination.

Among these higher alcohols, more preferred are oleyl alcohol, behenyl alcohol, myristyl alcohol, and octyldodecanol, and particularly preferred are oleyl alcohol and behenyl alcohol.

The amount of lipid composition in the adhesive composition is not limited. The content is preferably 0.1 mass% or more, more preferably 0.2 mass% or more, still more preferably 0.5 mass% or more to obtain the advantageous effects of the lipid composition. The content of the lipid composition in the adhesive composition is preferably 20 mass% or less, more preferably 15 mass% or less, still more preferably 10 mass% or less to give an appropriate adhesiveness to the resulting adhesive.

In the present embodiment, a lipid composition in the adhesive composition comprising a these components described above may be separately prepared in advance. Alternatively, the resin component, the components of the lipid composition, and optional components may be compounded to prepare an adhesive composition. In a preferred embodiment, the lipid composition prepared separately in advance is compounded with a resin component to prepare an adhesive composition to facilitate formation of a lamella structure.

[Resin component for forming adhesive]

The resin component of the present embodiment is not limited. For example, acrylic, rubber, silicone, and emulsion adhesives can be used for preparation of the adhesive composition of the present embodiment. Among these adhesives, more preferred are acrylic and rubber adhesives because the resin components of these adhesive can form radiation curable or hot-melt adhesive compositions described later.

The adhesive composition of the present embodiment can comprise a silicone adhesive in view of a reduction of stimuli to the skin in peeling of the adhesive.

Furthermore, the adhesive composition of the present embodiment can comprise an emulsion adhesive in preparation of a dermatological adhesive having a small environmental load.

Throughout the specification, the acrylic adhesive refers to an adhesive containing a polymer or copolymer including a structural unit derived from an acrylic acid ester. The rubber adhesive refers to an adhesive containing natural rubber, synthetic rubber, or a thermoplastic elastomer. The silicone adhesive refers to an adhesive containing a silicone polymer, such as polydimethylsiloxane. The emulsion adhesive refers to an acrylic adhesive, in particular, an adhesive containing an emulsion of water and a copolymer prepared through emulsion copolymerization of a monomer mixture containing methyl acrylate, (meth)acrylic ester having an alkyl group, and acrylic acid and/or methacrylic acid.

The adhesive composition of the present embodiment can be applied to base sheets and cured in any manner. For example, the adhesive composition of the present embodiment is preferably a hot-melt adhesive composition or a radiation curable adhesive composition, and is more preferably a radiation curable hot-melt adhesive composition. A hot-melt or radiation curable adhesive composition contains no organic solvent for dissolving the resin component in the composition, and thus ensures safety during operation, such as preparation and application of the composition and in use of the resulting adhesive on the skin.

In a hot-melt and/or radiation curable adhesive composition of the present embodiment, heat applied to such an adhesive composition seems to facilitate formation of the lamellar structure in the resulting adhesive. The present inventor believes that formation of the lamellar structure is facilitated because heat generated during mixing, application, and curing of the adhesive composition once dissolves the component capable of forming a lamellar structure (lipid composition) in the resin component of the adhesive composition, and the component (lipid composition) is deposited during formation of the adhesive.

The resin component of the adhesive composition of the present embodiment may be of any type that has adhesiveness to the skin and a shape retaining function due to the cohesive characteristics. The resin component can also be a resin used as a main component for conventional dermatological adhesives.

Examples of such a resin component includes natural rubber, synthetic rubber, synthetic resins, and thermoplastic elastomers.

Specific examples of the synthetic rubber include isoprene rubber (IR), styrene-butadiene rubber (SBR), butyl rubber (isobutylene-isoprene copolymer; IIR), and chloroprene rubber (CR).

Specific examples of the synthetic resins include vinyl acetate resins, ethylene-vinyl acetate copolymers, acrylate polymers, acrylate copolymers, silicone polymers, poly(vinyl alkyl ether), polyisobutylene (hereinafter, abbreviated to PIB), and vinyl acetate copolymers (hereinafter, abbreviated to EVA).

Examples of the thermoplastic elastomers include styrene-based thermoplastic elastomers, olefin thermoplastic elastomers, polyester thermoplastic elastomers, polyamide thermoplastic elastomers, urethane thermoplastic elastomers, vinyl chloride thermoplastic elastomers, and diene thermoplastic elastomers.

These resin components can be used alone or in combination.

In the case of use of a rubber adhesive comprising polyisobutylene among these synthetic resins as a skeletal component (main component) (for example, 35 mass% or more of the total mass of the adhesive composition), at least one of styrene-based elastomers, synthetic rubber, and synthetic resins is preferably used in combination as a component reinforcing the shape retaining function of the rubber adhesive. This is because the rubber adhesive achieves enhanced shape retention due to the cohesiveness of styrene-based elastomers, synthetic rubber, and synthetic resins. Among hydrophilic polymers described later, preferred are combinations of polyisobutylene with components such as pectin, gelatin, and guar gum, because the rubber adhesive has enhanced shape retention after absorption of water.

Preferred resin components are acrylic resins, such as (meth)acrylic ester polymers and (meth)acrylic ester copolymers, styrene-butadiene rubber, and styrene-based thermoplastic elastomers because of their commercial availability, adhesion to the skin, and safety. Acrylic adhesives or rubber adhesives are manufactured according to adhesive compositions containing these polymer compounds.

More preferred resin components are acrylic resins for forming hot-melt resin adhesive components and styrene-based thermoplastic elastomers for forming hot-melt resin adhesive components.

(Styrene-based thermoplastic elastomers)

Preferred examples of the resin component used in preparation of rubber adhesives from the adhesive composition of the present embodiment include styrene-butadiene rubber (SBR), hydrogenated styrene-butadiene rubber (HSBR), styrene-isoprene-styrene block copolymers (SIS), styrene-butadiene-styrene block copolymers (SBS), styrene-ethylene/propylene-styrene block copolymers (SEPS), styrene-ethylene/butylene-styrene block copolymers (SEBS), styrene-ethylene/ethylene/propylene-styrene block copolymers (SEEPS), and styrene-ethylene/butylene-olefin crystal block copolymers (SEBC).

Among these resin components, more preferred are hydrogenated styrene-butadiene rubber (HSBR) and styrene-isoprene-styrene block copolymers (SIS), and still more preferred is hydrogenated styrene-butadiene rubber (HSBR).

(Softening agent)

The rubber adhesive preferably contains a softening agent for imparting adhesiveness. Any softening agent can be used unless the object of the adhesive composition according to the present embodiment is not impaired. The softening agent can be a liquid or semi-solid resin.

Specific examples of the softening agent can include polyisobutylene, polyisoprene, polybutadiene, polybutene, styrene-isoprene rubber, ethylene-propylene rubber, styrene-ethylene-propylene rubber, and derivatives of hydrogenated products thereof. Among these softening agents, preferred is polyisobutylene. These softening agents can be used alone or in combination.

Although polyisobutylene can be used as a skeletal component of the adhesive composition, polyisobutylene can also be used as a softening agent in an adhesive composition containing the styrene-based thermoplastic elastomer as a resin component.

(Radiation curable resin)

Preferred examples of the resin component used in preparation of acrylic adhesives from the adhesive composition of the present embodiment include (meth)acrylic ester polymers (homopolymers of (meth)acrylic esters), and (meth)acrylate ester copolymers (copolymers of two or more (meth)acrylic esters, or copolymers of (meth)acrylic esters and other monomers). These radiation curable resins are preferably used. Ultraviolet light curable resins are more preferably used, and ultraviolet light curable hot-melt resins are still more preferably used.

Throughout the specification, the term "(meth)acrylic acid" refers to both "acrylic acid" and "methacrylic acid", and the term "(meth)acrylate" refers to both "acrylate" and "methacrylate".

The term "radiation curable resin" used as a resin component contained in the adhesive composition of the present embodiment refers to a resin composition that cures (or crosslinks) through irradiation with radiation rays, and contains at least one of a polymer, an oligomer, and a monomer as a component.

The "radiation rays" for the radiation curable resin can be any radiation that can give energy to the dermatological adhesive composition through irradiation to cause a curing (crosslinking) reaction of the dermatological adhesive composition. Examples of such radiation rays include electron beams, ultraviolet light, infrared light, laser beams, visible light, ionizing radiation rays (such as X rays, $\alpha$-rays, $\beta$-rays, and $\gamma$-rays), microwaves, and high frequency waves. Among these radiation rays, preferred are electron beams, visible light, and ultraviolet light, and more preferred is ultraviolet light. Preferred radiation curable resins are at least one selected from electron beam curable resins, visible light curable resins, and ultraviolet light curable resins. More preferred are ultraviolet light curable resins.

The curing reaction of the radiation curable resin can be performed by any mechanism. Preferred is a radical polymerization reaction, a cationic polymerization reaction, or a photodimerization reaction, and more preferred is a radical polymerization reaction or a photodimerization reaction.

A radiation curable resin preferably used is a polymer and/or a copolymer including a structural unit (repeating unit) derived from (meth)acrylate having an alkyl group having 1 to 20 carbon atoms, more preferably 2 to 10 carbon atoms, still more preferably 4 to 8 carbon atoms or a cycloalkyl group having 4 to 8 carbon atoms. The alkyl or cycloalkyl group may have a substituent. Examples of the substituent include halogen atoms, and hydroxy, aryl, alkoxy, phenoxy, epoxy, norbornyl, and adamantyl groups.

The radiation curable resins including a structural unit derived from (meth)acrylate are more preferably polymers and/or copolymers including a structural unit derived from butyl (meth)acrylate and/or a structural unit derived from 2-ethylhexyl (meth)acrylate, still more preferably polymers and/or copolymers including a structural unit derived from butyl acrylate, and/or a structural unit derived from 2-ethylhexyl acrylate.

Such polymers and/or copolymers having such structural units may be oligomers having a molecular weight smaller than those of the polymers.

The radiation curable resin has a mass average molecular weight Mw of preferably 1000 to 250000, more preferably 1000 to 200000. A radiation curable resin having an Mw within this range can provide a low-viscosity dermatological adhesive composition. The mass average molecular weight of the resulting dermatological adhesive composition increases through a curing reaction of the adhesive composition irradiated with radiation rays, providing an adhesive having a cohesive force. The Mw is determined by gel permeation chromatography (GPC).

The radiation curable resin can be in the form of a resin composition containing one or more polymerizable oligomers and/or polymerizable monomers for the polymer and/or the copolymer including a structural unit derived from (meth)acrylate. These polymerizable oligomers and/or polymerizable monomers are polymerized preferably by radical polymerization or cationic polymerization, more preferably radical polymerization.

The radiation curable resin may contain any polymerization initiator, additive, and solvent.

Any polymerization initiator known in the field of radiation curable resins can be used. Examples thereof include acetophenone initiators, benzophenone compounds, $\alpha$-ketoester compounds, and benzoin compounds.

The polymerization initiator is preferably bonded to the chain of the polymer or the oligomer as a component of the radiation curable resin rather than is contained in the radiation curable resin. Use of such a radiation curable resin having a polymerization initiator bonded to the chain of the polymer or the oligomer eliminates addition of the polymerization initiator, resulting in a safer dermatological adhesive composition.

Examples of such a radiation curable resin having a polymerization initiator introduced into the chain of the polymer or the oligomer include a trade name "UV-H" series made by KSM Co., Ltd., and a trade name "acResin" series made by BASF SE.

Another polymerization initiator may further be bonded to the radiation curable resin having a polymerization initiator already bonded thereto. Alternatively, a polymerization initiator bonded to the radiation curable resin may be replaced with another polymerization initiator.

The resin component can be used in any content.

For example, in the case where the resin component is a suitable styrene-based thermoplastic elastomer, the content is preferably 2 to 35 mass%, more preferably 5 to 20 mass% of the total mass of the adhesive composition.

For example, in the case where the resin component is a suitable radiation curable resin, the content is preferably 30 to 95 mass%, more preferably 30 to 90 mass%, still more preferably 40 to 80 mass% of the total mass of the adhesive composition.

An adhesive composition containing the resin component in an amount in the range described above can be formed into an adhesive having good cohesiveness.

(Reactive diluent)

In the case where the resin component is a radiation curable resin, the adhesive composition of the present embodiment more preferably contains a diluent reactive with the radiation curable resin.

Throughout the specification, the term "reactive diluent" refers to a liquid component that is reactive with the radiation curable resin and reduces the viscosity of the adhesive composition or the resin content in the adhesive composition.

A radiation curable adhesive composition containing such a reactive diluent facilitates processes, such as application to base sheets, and can provide an adhesive having good cohesiveness and low bleeding characteristics because the reactive diluent is crosslinked through a reaction with the radiation curable resin after irradiation with radiation rays.

The curing reaction of the adhesive composition containing the reactive diluent proceeds more readily than in an adhesive composition containing a softening agent without the reactive diluent, resulting in a reduction in the dose of radiation rays. For this reason, production efficiency can also be enhanced.

The reactive diluent preferably contains a compound having a (meth)acryloyl group as a functional group. Throughout the specification, the term "(meth)acryloyl group" refers to both an "acryloyl group" ($H_2C=CH-C(=O)-$) and a "methacryloyl group" ($H_2C=C(CH_3)-C(=O)-$).

The reactive diluent used in the present embodiment can be added to a commercially available radiation curable resin.

The preferable number of (meth)acryloyl groups of the compound having a (meth)acryloyl group is, though it is not limited to, 1 to 3, and the reactive diluent preferably has 1 to 3 (meth)acryloyl groups per molecule. The preferable molecular weight of the compound having a (meth)acryloyl group is, though, it is not limited to, 1000 or less, more preferably 800 or less, still more preferably 600 or less.

Examples of the compound having a (meth)acryloyl group used as a reactive diluent include (meth)acrylate compounds and (meth)acrylamide compounds.

Throughout the specification, the term "(meth)acrylate compound" refers to a (meth)acrylic ester compound having a linear or branched alkyl group or a cycloalkyl group and having optional substituents in the alkyl group and the cycloalkyl group.

The term "(meth)acrylamide compound" refers to an amide compound having a (meth)acryloyl group, and having an optional substituent in an amino group of (meth)acrylamide. The term "(meth)acrylic" refers to both "acrylic" and "methacrylic".

The "(meth)acrylate compound" and the "(meth)acrylamide compound" can have any substituent. Examples of the substituent include hydroxy, alkoxy, and phenoxy groups.

The "compound having a (meth)acryloyl group" used as the reactive diluent is preferably one or more compounds selected from acryloylmorpholine, hydroxyethylacrylamide, phenoxyethyl acrylate, 1,3-dioxolane acrylate, isobornyl acrylate, poly(ethylene glycol) diacrylate, propylene oxide (PO)-modified trimethylolpropane triacrylate, and methoxypolyethylene glycol acrylate.

The "compound having a (meth)acryloyl group" is more preferably one or more compounds selected from acryloylmorpholine, polyethylene glycol diacrylate, and PO-modified trimethylolpropane triacrylate. The "compound having a (meth)acryloyl group" is still more preferably a polyfunctional compound having three or more functional groups.

A preferred (meth)acrylate compound usable as a reactive diluent is a (meth)acrylate having a trimethylolpropane skeleton having a quaternary carbon atom and having a symmetricity about the quaternary carbon. Examples of the (meth)acrylate having a trimethylolpropane skeleton can include trimethylolpropane tri(meth)acrylate, ditrimethylolpropane tetra(meth)acrylate, and alkylene oxide-modified trimethylolpropane tri(meth)acrylate.

The reactive diluent has a viscosity of preferably 1000 mPa·s or less, more preferably 500 mPa·s or less at 25°C. The lower limit is preferably 1 mPa·s.

A reactive diluent having a viscosity in this range at 25°C can facilitate mixing with the radiation curable resin and processes, such as application.

Throughout the specification, the viscosity (steady-flow viscosity) of the reactive diluent is measured at 25°C with a monocylindrical rotational viscometer in accordance with JIS Z8803.

The preferable content of the reactive diluent in adhesive composition is 1 to 30 mass% of the total mass of the adhesive composition. The content of the reactive diluent is preferably 1 to 20 mass%, more preferably 2 to 15 mass%, still more preferably 2 to 8 mass% of the total mass of the adhesive composition to effectively reduce the viscosity of the adhesive and provide a dermatological adhesive having high adhesiveness.

The total content of the radiation curable resin and the reactive diluent is preferably 50 to 100 mass%, more preferably 60 to 85 mass%, still more preferably 70 to 85 mass% of the total mass of the adhesive composition.

The content of the reactive diluent in the range described above can reduce the viscosity of the dermatological adhesive composition to facilitate processes, such as application to base sheets. Such dermatological adhesive can achieve low bleeding characteristics and good cohesiveness.

(Functional group-free acrylic polymer)

In the case where the radiation curable resin is used as a resin component, the adhesive composition of the present embodiment preferably contains a reactive diluent and a functional group-free acrylic polymer, which reduces the viscosity of the adhesive composition.

An adhesive composition containing a component for reducing the viscosity, such as a softening agent (plasticizer), but not containing a reactive diluent may cause bleeding from the adhesive or impair the cohesive characteristics of the adhesive. The present inventor, however, has found that unexpectedly, the reactive diluent in combination with a functional group-free acrylic polymer can effectively reduce the viscosity of the adhesive composition, and thus enhance the adhesiveness of the adhesive prepared from the adhesive composition.

Namely, the adhesive composition of the present embodiment preferably comprises a radiation curable resin as a resin component, a diluent reactive with the radiation curable resin, a functional group-free acrylic polymer, and a lamellar structure comprising a lipid composition. Also preferred is use of, instead of a lamellar structure comprising a lipid composition, a lipid composition comprising at least one selected from the group consisting of sphingolipids, sterols, and glycerol fatty acid esters, instead of the lamellar structure formed of a lipid composition; and at least one selected from the group consisting of higher alcohols, acylated lactic acids, and acylated lactates.

The dermatological adhesive composition also preferably contains a hydrophilic polymer described later, or may contain a tackifier, a filler, a pH adjuster, active ingredients, and a plasticizer described later.

The term "acrylic polymer" in the functional group-free acrylic polymer refers to a polymer or copolymer mainly including 50 mol % or more of a structural unit derived from acrylate and/or methacrylate. The acrylic polymer includes a structural unit derived from acrylate and/or methacrylate in an amount of preferably 60 to 100 mol %, more preferably 70 to 100 mol %, still more preferably 80 to 100 mol %. The proportion (mol %) of the structural unit derived from acrylate and/or methacrylate in the acrylic polymer can be determined with a nuclear magnetic resonance (NMR) spectrometer.

The term "functional group-free" of the functional group-free acrylic polymer indicates that the acrylic polymer substantially does not have any functional group other than the acryloyl group. Examples of the other functional groups in this case include -OH, -COOH, epoxy, and alkoxysilyl groups.

The functional group-free acrylic polymer has a mass average molecular weight (Mw) of preferably 1000 to 9000, more preferably 1500 to 8000, still more preferably 2000 to 6000 in view of an effective reduction in viscosity of the adhesive composition and readiness in preparation of an adhesive having a cohesive force. Throughout the specification, the value of the Mw is determined with a gel permeation chromatography (GPC).

The functional group-free acrylic polymer is preferably liquid at normal temperature (20 to 30°C) in view of an effective reduction in viscosity of the adhesive composition. A functional group-free liquid acrylic polymer at normal temperature has preferably 90% or more, more preferably 95% or more, still more preferably 98% or more solid content.

The functional group-free acrylic polymer has a viscosity of preferably 300 to 10000 mPa·s, more preferably 400 to 6000 mPa·s, still more preferably 500 to 5000 mPa·s at 25°C. Throughout the specification, the viscosity (steady-flow viscosity) of the functional group-free acrylic polymer is measured at 25°C with a monocylindrical rotational viscometer in accordance with JIS Z8803.

The functional group-free acrylic polymer has a glass transition temperature (Tg) preferably -100 to -20°C, more preferably -90 to -40°C, still more preferably -80 to -60°C in view of an effective reduction in viscosity of the adhesive composition. The Tg is determined with a differential scanning calorimeter (DSC).

The content of the functional group-free acrylic polymer is not limited. The content is preferably 1 to 20 mass%, more preferably 5 to 18 mass%, still more preferably 7.5 to 15 mass% of the total mass of the adhesive composition. The combination of the functional group-free acrylic polymer used in a content in the range described above and the reactive diluent can effectively reduce the viscosity of the adhesive composition and effectively enhance the cohesiveness of the resulting adhesive.

(Hydrophilic polymer)

The adhesive composition of the present embodiment may contain a hydrophilic polymer that can be used as a hydrocolloid component. Any hydrophilic polymer, such as natural, semi-synthetic, or synthetic hydrophilic polymer, can be used. The "semi-synthetic", which is also called partially chemically synthetic, refers to a state generated from chemical synthesis using a starting material formed of a compound isolated from a natural resource, such as a plant material, a microorganism, or a cell culture product.

Specific examples of the natural hydrophilic polymer include plant-derived polymers, such as gum arabic, tragacanth gum, galactan, guar gum, carob gum, karaya gum, carrageenan, pectin, agar, and starches (such as rice, corn, potato, and wheat starches); microorganism-derived polymers, such as xanthan gum, dextrin, dextran, succionoglucan, mannan, locust bean gum, and pullulan; and animal-derived polymers, such as casein, albumin, and gelatin.

Specific examples of the semi-synthetic hydrophilic polymer include starch polymers (such as carboxymethyl starch and methylhydroxypropyl starch); cellulose polymers (such as methyl cellulose, ethyl cellulose, methylhydroxypropyl cellulose, hydroxyethyl cellulose, cellulose sodium sulfate, hydroxypropyl cellulose, carboxymethyl cellulose, carboxymethyl cellulose sodium); and alginic polymers (such as sodium alginate and propylene glycol alginate).

Specific examples of the synthetic hydrophilic polymer include vinyl polymers (such as poly(vinyl alcohol), poly(vinyl methyl ether), poly(vinylpyrrolidone), and carboxyvinyl polymer); acrylic polymers (such as sodium polyacrylate and polyacrylamide); and polyethyleneimines.

These hydrophilic polymers can be used alone or in combination.

Among these hydrophilic polymers, preferred is use of one or more polymers selected from carboxymethyl cellulose sodium, pectin, karaya gum, mannan, guar gum, locust bean gum, and gelatin, and more preferred is use of one or more polymers selected from carboxymethyl cellulose sodium, pectin, mannan, and locust bean gum.

In the case of an adhesive prepared with the adhesive composition of the present embodiment containing the hydrophilic polymer, the adhesive applied to the skin can absorb sweat on the skin or exudates from wounds to alleviate skin rashes and itches caused by heat and moisture under the adhesive. Such an adhesive can reduce stimuli to the skin caused by skin maceration, and can be applied to the skin for a long time.

The content of the hydrophilic polymer is preferably 2.5 to 50 mass%, more preferably 5 to 35 mass%, still more preferably 7 to 25 mass% of the total mass of the adhesive composition in view of the moisture absorbing ability of the adhesive and a reduction in stimuli of the adhesive to the skin.

(Emulsion)

Examples of resin components suitable for preparation of emulsion adhesives with the adhesive composition of the present embodiment include methyl acrylate, (meth)acrylic esters having an alkyl group, and copolymer emulsions prepared through emulsion copolymerization of monomer mixtures containing acrylic acid and/or methacrylic acid. Among these resin components, preferred are copolymers prepared through emulsion copolymerization of monomer mixtures containing (meth)acrylic ester in a ratio of 2-ethylhexyl acrylate:methyl acrylate:methyl methacrylate=60 to 80:15 to 30:1 to 8.

Such emulsion adhesives are prepared through the following steps, for example. 2-Ethylhexyl acrylate (73 parts), methyl acrylate (20 parts), methyl methacrylate (3 parts), acrylic acid (1 part), and methacrylic acid (3 parts) are prepared. To the total amount of these monomers (100 parts), 0.1 parts of octyl thioglycolate and 2 parts of a reactive ammonia-neutralized anionic surfactant (made by Dai-ichi Kogyo Seiyaku Co., Ltd., trade name "Aqualon KH-10") are dissolved in deionized water (32 parts). The resulting solution is added to the monomers, and are emulsified with stirring to prepare an emulsified product. The emulsified product is put in a dropping funnel. Deionized water (75 parts) and an anionic emulsifier polyoxyethylene alkyl ether sulfuric acid ester salt (made by NIPPON NYUKAZAI CO., LTD., trade name "RA-9607") (0.2 parts) are charged in a four-necked flask equipped with a stirrer, a cooling tube, a thermometer, and the dropping funnel. The inside of the flask is purged with nitrogen gas. The inner temperature is raised to 80°C with stirring, and an aqueous solution of 3% potassium persulfate (content: 0.1 parts) is added. After five minutes, the emulsified product is added dropwise from the dropping funnel, and concurrently an aqueous solution of 3% potassium persulfate (content: 0.35 parts) is added dropwise from another port over three hours. After 60 minutes has passed from the end of addition of the emulsified product, three aliquots of 2 parts of an aqueous solution of 1% t-butyl hydroperoxide and 2 parts of an aqueous solution of 1% rongalit are added at an interval of 30 minutes while the inner temperature is kept at 80°C. The mixture is aged at 80°C for 60 minutes while stirring is continued. The reaction product is cooled, and is neutralized with aqueous ammonia to prepare an acrylic copolymer emulsion (solid content: 45%).

(Silicone)

The preferred resin component of silicone adhesive of the present embodiment is, though it is not limited to, any one of the resin components of addition reaction, peroxidation reaction, and condensation reaction types can be used. Among them, the more preferred are silicone resins of addition reaction types. Preferred silicone resins of addition reaction types are those prepared through addition reaction (hydrosilylation reaction) of organopolysiloxane having an alkenyl group bonded to a silicon atom (alkenyl group-containing organopolysiloxane) and organopolysiloxane having a hydrosilyl group (Si-H) (organohydrogen polysiloxane) in the presence of a platinum compound catalyst, such as chloroplatinic acid. The silicone resin of addition reaction type can have a crosslinking density controlled by varying the amount of organohydrogenpolysiloxane and the amount of the hydrosilyl group (Si-H) in the organohydrogenpolysiloxane molecule used in the synthesis. The control of the crosslinking density can facilitate the control of the hardness and the adhesiveness of the silicone resin. These silicone resins can be used alone or in combination. Preferred silicone resins are cured through crosslinking. The silicone resin can be cured by any method, and can be readily crosslinked by heating.

(Other components)

The adhesive composition according to the present embodiment may contain a plasticizer, a tackifier, a filler, a pH adjuster, and active ingredients when necessary as long as they do not impair the object of the adhesive composition according to the present embodiment.

Any plasticizer can be used unless the object of the adhesive composition according to the present embodiment is impaired. Examples of the plasticizer include mineral oils, such as liquid paraffin and naphthene oil; vegetable oils, such as olive oil, cater oil, and palm oil; animal oils, such as lanolin, turtle oil, and beeswax; and synthetic oils, such as silicone oils and ester oils.

Examples of the silicone oils include those modified with $-O(EO)_n-$ (polyether), $-NH_2$ (amino), $-OH$ (hydroxyl), $-CH_2OH$ (carbinol), and $-COOH$ (carboxyl) groups. Among these silicone oils, particularly preferred is polyether ($-O(EO)_n-$) modified silicone.

These plasticizers can be used alone or in combination. The plasticizer is preferably contained in a rubber adhesive.

Known tackifiers can be used unless the object of the adhesive composition according to the present embodiment is impaired. Examples of the tackifier include petroleum resins, such as alicyclic saturated hydrocarbon resins prepared through high pressure hydrogenation of aliphatic copolymers, aromatic copolymer, aliphatic·aromatic copolymers, alicyclic copolymers, and aromatic petroleum resins; and aliphatic hydrocarbon resins prepared through polymerization of aliphatic hydrocarbons, such as rosin derivatives, terpene resins, coumarone-indene resins, and 1,3-pentadiene. Among these resins, preferred are alicyclic saturated hydrocarbon resins.

Any known filler can be used unless the object of the adhesive composition according to the present embodiment is impaired. Examples of the filler include silica, silylated silica, alumina, and talc. Silylated silica preferably used is those having a silanol group capped with a trialkylsilyl group.

It may be desired in some cases that the dermatological adhesive prepared from the adhesive composition according to the present embodiment have reduced stickiness in terms of workability. To achieve such a dermatological adhesive, the adhesive composition preferably contains polyether-modified silicone (hereinafter, referred to as PE-modified silicone).

In this case, the silylated silica is preferably used as a filler to prevent bleed of the PE-modified silicone from the dermatological adhesive.

Any known pH adjuster can be used unless the object of the adhesive composition according to the present embodiment is impaired. Examples of the pH adjuster include buffer solutions of citric acid, anhydrous, alkali metal hydroxides, and organic acids.

Any active ingredients can be used unless the object of the adhesive composition according to the present embodiment is impaired. Examples of the active ingredients include drugs, such as physiological activators, antibacterial agents, anti-inflammatory analgesics, steroidal agents, anesthetic agents, antifungal agents, bronchodilators, antitussive drugs, coronary vasodilators, antihypertensive agents, hypotensive diuretics, antihistamines, sedative-hypnotics, tranquilizers, vitamin preparations, sex hormonal agents, antidepressants, cerebral circulation improvers, antiemetics, and antitumor medicines. These drugs systemically or locally exhibit their effects through dermal absorption, or locally exhibit their effects on the place in situ to which the adhesive is applied.

The adhesive composition according to the present embodiment can be used as a dermatological adhesive for a patch.

The patch includes a base sheet, and an adhesive layer comprising the adhesive composition according to the present embodiment and disposed on the base sheet. An adhesive composition containing polyisobutylene as a skeletal component can not only be used as a dermatological adhesive in a patch, but also be used alone as a dermatological adhesive without base sheet.

Specific examples of preferred patches include wound dressings applied to living bodies, surgical tapes, tapes for fixing catheters and medical drip tubes, patches for ostomy appliances, poultices, patches for fixing electrodes for electrocardiographs and magnetic therapeutic apparatuses, and patches for skin care and beauty.

Since the dermatological adhesive composition according to the present embodiment provides high recovery of the TEWL, the dermatological adhesive composition according to the present embodiment can be suitably used in wound dressings, patches for ostomy appliances, surgical tapes, and tapes for fixing catheters and medical drip tubes, which are applied to the skin for a relatively long time.

(Base sheet)

A film, a foam, or a fabric (such as non-woven fabrics, woven fabrics, or knitted fabrics) can be the base sheet. Among these materials, preferred are films and non-woven fabrics in view of their flexibility, stretching characteristics, and appropriate steam permeability.

Specific examples of materials for the films and the non-woven fabrics can include polyurethane, polyester, polyamide, polyethylene, polypropylene, acrylic polymers, polyolefin elastomers, and olefin copolymers. Among these materials, preferred are polyurethane, polyester, polyolefin elastomers, olefin copolymers, and polyamides.

The base sheet has a thickness of preferably 5 to 150 $\mu$m, particularly preferably 10 to 100 $\mu$m.

The adhesive composition of the present embodiment has been described. One example of a preferred aspect of the present embodiment also include a dermatological adhesive composition comprising 2 to 35 mass% styrene-based elastomer, 0.1 to 20 mass% lipid composition, and 2.5 to 50 mass% hydrophilic polymer, and a dermatological adhesive composition comprising 0.4 to 15 mass% styrene-based elastomer, 0.5 to 10 mass% lipid composition, 35 to 50 mass% hydrophilic polymer, 20 to 30 mass% softening agent, and 10 to 25 mass% tackifier. Furthermore, another example of a preferred aspect of the adhesive composition according to the present embodiment can also include a dermatological adhesive composition comprising 30 to 95 mass% radiation curable resin, 0.1 to 20 mass% lipid composition, and 2.5 to 50 mass% hydrophilic polymer, and a dermatological adhesive composition comprising 60 to 85 mass% radiation curable resin, 0.5 to 10 mass% lipid composition, and 5 to 20 mass% hydrophilic polymer.

The adhesive composition of the present embodiment can also have the following aspects 1 to 19.

[Aspect 1] A dermatological adhesive composition comprising a resin component for forming an adhesive, and a lamellar structure formed of a lipid composition.

[Aspect 2] The dermatological adhesive composition according to aspect 1, wherein the lamellar structure is formed of a lipid composition containing a sphingolipid and/or a sterol.

[Aspect 3] The dermatological adhesive composition according to aspect 1 or 2, wherein the lamellar structure is formed of a lipid composition comprising at least one selected from sphingolipids, sterols, and surfactants; and a higher alcohol.

[Aspect 4] The dermatological adhesive composition according to aspects 1 to 3, wherein the surfactant is at least one selected from the group consisting of glycerol fatty acid esters, acylated lactic acids and/or salts thereof, polyoxyalkylene alkyl ethers, polyoxyalkylene glycol fatty acid esters, and sorbitan fatty acid esters and alkylene glycol adducts thereof.

[Aspect 5] The dermatological adhesive composition according to any one of aspects 1 to 4, wherein the lamellar structure is formed of a lipid composition comprising a glycerol fatty acid ester, and an acylated lactic acid and/or a salt thereof.

[Aspect 6] The dermatological adhesive composition according to any one of aspects 1 to 5, wherein the lamellar structure is formed of a lipid composition comprising polyoxyalkylene alkyl ether or polyoxyalkylene glycol fatty acid ester as a surfactant.

[Aspect 7] The dermatological adhesive composition according to any one of aspects 1 to 6, wherein the lamellar structure is formed of a lipid composition comprising at least one selected from the group consisting of sphingolipids, sterols, and surfactants in a total amount of 5 to 80 mass%, and higher alcohols in a total amount of 10 to 50 mass%.

[Aspect 8] The dermatological adhesive composition according to any one of aspects 1 to 7, wherein the lamellar structure is formed of a lipid composition comprising at least one selected from the group consisting of sphingolipids, sterols, and glycerol fatty acid esters in a total amount 5 to 80 mass%, and higher alcohols in a total amount of 10 to 50 mass%.

[Aspect 9] The dermatological adhesive composition according to any one of aspects 1 to 8, wherein the resin component for forming an adhesive is an acrylic resin serving as a resin component for forming a hot-melt adhesive.

[Aspect 10] The dermatological adhesive composition according to any one of aspects 1 to 8, wherein the resin component for forming an adhesive is a styrene-based thermoplastic elastomer serving as a resin component for forming a hot-melt adhesive.

[Aspect 11] The dermatological adhesive composition according to any one of aspects 1 to 10, further comprising a hydrophilic polymer.

[Aspect 12] A dermatological adhesive composition comprising a resin component for forming an adhesive; and a lipid composition comprising at least one selected from sphingolipids and sterols, and at least one selected from the group consisting of higher alcohols and surfactants.

[Aspect 13] The dermatological adhesive composition according to aspect 12, wherein the surfactant is at least one selected from the group consisting of glycerol fatty acid esters, acylated lactic acids and/or salts thereof, polyoxyalkylene alkyl ethers, polyoxyalkylene glycol fatty acid esters, and sorbitan fatty acid esters and alkylene glycol adducts thereof.

[Aspect 14] The dermatological adhesive composition according to aspect 12 or 13, wherein the lipid composition comprises at least one selected from the group consisting of sphingolipids, sterols, and surfactants in a total amount of 5 to 80 mass%; and higher alcohols in a total amount of 10 to 50 mass%.

[Aspect 15] The dermatological adhesive composition according to any one of aspects 12 to 14, wherein the lipid composition comprises at least one selected from the group consisting of sphingolipids, sterols, and glycerol fatty acid esters in a total amount of 5 to 80 mass%; and higher alcohols in a total amount of 10 to 50 mass%.

[Aspect 16] The dermatological adhesive composition according to any one of aspects 12 to 15, wherein the resin component for forming an adhesive is an acrylic resin serving as a resin component for forming a hot-melt adhesive.

[Aspect 17] The dermatological adhesive composition according to any one of aspects 12 to 15, wherein the resin component for forming an adhesive is a styrene-based thermoplastic elastomer serving as a resin component for forming a hot-melt adhesive.

[Aspect 18] The dermatological adhesive composition according to any one of aspects 11 to 17, further comprising a hydrophilic polymer.

[Aspect 19] A dermatological patch including a base sheet, and an adhesive layer formed of the dermatological adhesive composition according to any one of aspects 1 to 18 and disposed on the base sheet.

In aspect 3 or 14, the surfactant may be a non-ionic or anionic surfactant.

Examples

The adhesive composition according to the present embodiment will now be described in more detail by way of Examples. Examples described below are illustrative of the adhesive composition according to the present embodiment, and should not be construed to limit the scope of the adhesive composition according to the present embodiment.

Lipid compositions A to I containing components shown in Table 1 were used in Examples and Comparative Examples. TIC-001 made by TAKASAGO INTERNATIONAL CORPORATION was used as Ceramide 2.

[Table 1]

| Limpid composition (mass proportion) | | A | B | C | D | E | F | G | H | I |
|---|---|---|---|---|---|---|---|---|---|---|
| Sphingolipid | Ceramide 2 | 10 | - | - | 40 | 10 | 20 | 50 | 30 | 50 |
| Sterol | Cholesterol | - | 10 | | 30 | - | - | - | - | - |
| Higher alcohols | 1-Docosanol | 45 | 45 | 45 | - | 45 | - | - | - | - |
| | Oleyl alcohol | - | - | - | - | - | - | - | - | - |
| | Myristyl alcohol | - | - | - | 30 | - | - | 30 | - | 30 |
| | Octyldodecanol | - | - | - | - | - | 30 | - | 20 | - |
| Surfactants | Polyglyceryl-10 pentastearate | 34.2 | 34.2 | 34.2 | - | - | - | - | - | - |
| | Pentaerythrityl tetraisostearate | - | - | - | - | - | - | - | - | - |
| | Sorbitan monostearate | - | - | - | - | - | - | - | 50 | - |
| | Polyethylene glycol (45 E.O.) monostearate | - | - | - | - | 34.2 | - | - | - | - |
| | Na stearoyllactate | 10.8 | 10.8 | 10.8 | - | 10.8 | - | - | - | - |
| | POE(9) lauryl ether | - | - | - | - | - | - | 20 | - | - |
| | POE(2) stearyl ether | - | - | - | - | - | - | - | - | - |

* "-" in the table indicates "not contained" (the same is also true in the following tables).

** The surfactants have the following HLB values:

Polyglyceryl-10 pentastearate: 3.5

Pentaerythrityl tetraisostearate: 6.0

Sorbitan monostearate: 6.0

Polyethylene glycol (45 E.O.) monostearate: 18

POE(9) lauryl ether: 14.5

POE(2) stearyl ether: 8.0

<Example 1>

Hydrogenated styrene-butadiene rubber (HSBR; made by JSR Corporation) as a styrene-based thermoplastic elastomer, polyisobutylene (Himol 6H made by JX Nippon Oil & Energy Corporation) as a softening agent, and liquid paraffin (Hicall K-350 made by KANEDA Co., Ltd.) as a plasticizer were placed in a pressure kneader, and mixed under pressure to yield a sufficiently homogeneous mixture.

Carboxymethylcellulose sodium (CMC·Na) and pectin as hydrophilic polymers, a tackifier (ARKON P-100, commercially available alicyclic saturated hydrocarbon resin from ARAKAWA CHEMICAL INDUSTRIES, LTD.), and Lipid composition A were added, and mixed to prepare a homogeneous adhesive composition of Example 1.

The adhesive composition of Example 1 was prepared where the hydrogenated styrene-butadiene rubber was 11 mass%, the polyisobutylene 26 mass%, the liquid paraffin 5 mass%, CMC·Na 21 mass%, pectin 22 mass%, the tackifier 14 mass%, and Lipid composition A 1 mass%.

<Example 2>

An adhesive composition of Example 2 was prepared in a similar way to Example 1 except that the content of CMC·Na was 19 mass% and the content of Lipid composition A was 3 mass%.

<Example 3>

An adhesive composition of Example 3 was prepared where the hydrogenated styrene-butadiene rubber was 7.1 mass%, the polyisobutylene 23.5 mass%, the liquid paraffin 3.1 mass%, CMC·Na 21.9 mass%, pectin 21.5 mass%, the tackifier 21.9 mass%, and Lipid composition A 1 mass%.

<Example 4>

An adhesive composition of Example 4 was prepared in a similar way to Example 3 except that the content of CMC·Na was 24.2 mass% and the content of pectin was 19.2 mass%.

<Example 5>

An adhesive composition of Example 5 was prepared where the hydrogenated styrene-butadiene rubber was 7.1 mass%, the polyisobutylene was 23.5 mass%, the liquid paraffin was 3.1 mass%, CMC·Na was 19.9 mass%, the pectin was 19.5 mass%, the tackifier was 23.9 mass%, and Lipid composition A was 3 mass%.

<Example 6>

An adhesive composition of Example 6 was prepared where the hydrogenated styrene-butadiene rubber was 13.4 mass%, the polyisobutylene was 26.5 mass%, the liquid paraffin was 5.8 mass%, CMC·Na 19.9 was mass%, the pectin 19.5 was mass%, the tackifier was 13.9 mass%, and Lipid composition A was 1 mass%.

<Example 7>
An adhesive composition of Example 7 was prepared in a similar way to Example 3 except that Lipid composition F (1 mass%) was compounded instead of Lipid composition A used in preparation of the adhesive composition of Example 3.

<Example 8>

An adhesive composition of Example 8 was prepared in a similar way to Example 3 except that Lipid composition G (1 mass%) was compounded instead of Lipid composition A used in preparation of the adhesive composition of Example 3.

<Example 9>

An adhesive composition of Example 9 was prepared in a similar way to Example 3 except that Lipid composition H (1 mass%) was compounded instead of Lipid composition A used in preparation of the adhesive composition of Example 3.

<Comparative Example 1>

An adhesive composition of Comparative Example 1 was prepared in a similar way to Example 1 except that the content of CMC·Na was 21.7 mass%, and Ceramide 2 (TIC-001 made by TAKASAGO INTERNATIONAL CORPORATION) (0.3 mass%) was compounded instead of Lipid composition A.

(Evaluation of Examples 1 to 9 and Comparative Example 1)

In the adhesive compositions of Examples 1 to 9 and Comparative Example 1, the adhesiveness and the TEWL recovery rate were determined by the following methods.
The adhesive compositions prepared in Examples 1 to 9 and Comparative Example 1 were each applied to a base sheet, for example, a polyethylene film (thickness: 70 μm) at 100°C with a laminator such that the thickness was 1.5 mm. Adhesives (adhesive layers) formed of the adhesive compositions of Examples 1 to 9 and Comparative Example 1 were disposed on the base sheet sheets in such a manner to prepare patches of Examples 1 to 9 and Comparative Example 1. The patches were aged at 60°C for five days before evaluation of the patches.

(Adhesiveness to skin)

In the patches formed of the adhesive compositions of Examples and Comparative Example above, the adhesiveness (peel force) was measured with a tensile tester (product name "INSTRON 5564" made by Instron Corporation) at a tensile rate of 1000 mm/min in accordance to JIS Z0237. The test pieces of the patches for the measurement were applied to the bellies of subjects or adherents. Each test piece had a width of 1 inch (25.4 mm) and a length of 50 mm.

The adhesiveness to the skin was measured after one day or three days from the application of the patches to the bellies of the subjects.

In the measurement of the adhesiveness, the state of the test piece in peeling from the adherent (peeling mode) was determined whether the peeling mode was "interfacial peeling" in which the adhesive peeled off from the adherent at the interface or "cohesive failure" in which the adhesive broke.

(Adhesiveness to Bakelite plate)

In the patches formed of the adhesive compositions of Examples and Comparative Example, the adhesiveness (peel force) was measured with a tensile tester (product name "INSTRON 5564" made by Instron Corporation) at a tensile rate of 300 mm/min in accordance to JIS Z0237. The test pieces of the patches for the measurement were applied to Bakelite plates as adherents. Each test piece had a width of 1 inch (25.4 mm) and a length of 100 mm.

During the measurement of the adhesiveness, the state of the test piece in peeling from the adherent (peeling mode) was determined whether the peeling mode was "interfacial peeling," in which the adhesive peeled off from the adherent at the interface or "cohesive failure," in which the adhesive broke.

(TEWL recovery rate)

The TEWL value was measured with a moisture loss measuring apparatus (product name "Cyclone moisture evaporation monitor AS-CT1" made by ASAHIBIOMED Ltd.). In this test, the patches of Examples were applied to the bellies of subjects, and the TEWL recovery rate of each patch was measured. A higher TEWL value for evaluation, that is, a larger moisture loss from the skin than normal indicates that the skin barrier function reduced.

The skin was treated with acetone, and was subjected to a tape stripping treatment to the skin rough. The TEWL of the rough skin immediately after this treatment was measured, which was the most damaged skin ($TEWL_{A0}$). The TEWL of normal skin ($TEWL_{B0}$) was subtracted from the TEWL of the rough skin to calculate the corrected value of the TEWL of the normal skin. In contrast, a patch was applied to the rough skin portion. After some days (X days) had passed (such as Day 1 or 3), the TEWL was measured ($TEWL_{AX}$). The TEWL of the normal skin ($TEWL_{BX}$) on Day X was subtracted from $TEWL_{AX}$ to calculate the corrected value of the normal skin.

Based on the numeric values (TEWL values) obtained in such a manner, the recovery rate was determined according to the following expression. Namely, a smaller difference in TEWL between the portion of the rough skin from which the patch was removed after appropriate days had passed (on Day X) and the normal skin (in other words, the skin barrier function of the rough skin closer to that of the normal skin) indicates a higher TEWL recovery rate and a higher effect of recovering the skin barrier function.

[Expression 2]

$$\text{TEWL recovery rate} = \left(1 - \frac{TEWL_{AX} - TEWL_{BX}}{TEWL_{A0} - TEWL_{B0}}\right) \times 100 \, (\%)$$

$TEWL_{A0}$ : TEWL of rough skin on Day 0 (patch was not applied)
$TEWL_{B0}$ : TEWL of normal skin on Day 0 (patch was not applied)
$TEWL_{AX}$ : TEWL on Day X after patch was applied to rough skin
$TEWL_{BX}$ : TEWL of normal skin on Day X (patch was not applied)

(Examination of maltese cross image)

To confirm a lamellar structure in the adhesive layer formed of each adhesive composition, maltese cross images, which is inherent in the lamella structure, in adhesive layers formed of adhesive compositions for observation were examined with a polarizing microscope. The adhesive compositions for observation were prepared with the components of the adhesive compositions of Examples 1 to 9 and Comparative Example 1 not containing the tackifier, pectin, and CMC·Na.

The results in the measurement of the adhesive compositions and patches of Examples 1 to 9 and Comparative Example 1 are shown in Table 2.

[Table 2]

| | | Example 1 | Example 2 | Example 3 | Example 4 | Example 5 | Example 6 | Example 7 | Example 8 | Example 9 | Comparative Example 1 |
|---|---|---|---|---|---|---|---|---|---|---|---|
| Composition (mass%) | HSBR | 11 | 11 | 7.1 | 7.1 | 7.1 | 13.4 | 7.1 | 7.1 | 7.1 | 11 |
| | Liquid paraffin | 5 | 5 | 3.1 | 3.1 | 3.1 | 5.8 | 3.1 | 3.1 | 3.1 | 5 |
| | Polyisobutylene | 26 | 26 | 23.5 | 23.5 | 23.5 | 26.5 | 23.5 | 23.5 | 23.5 | 26 |
| | Tackifier | 14 | 14 | 21.9 | 21.9 | 23.9 | 13.9 | 21.9 | 21.9 | 21.9 | 14 |
| | Pectin | 22 | 22 | 21.5 | 19.2 | 19.5 | 19.5 | 21.5 | 21.5 | 21.5 | 22 |
| | CMC·Na | 21 | 19 | 21.9 | 24.2 | 19.9 | 19.9 | 21.9 | 21.9 | 21.9 | 21.7 |
| | Lipid composition A | 1 | 3 | 1 | 1 | 3 | 1 | - | - | - | - |
| | Lipid composition F | - | - | - | - | - | - | 1 | - | - | - |
| | Lipid composition G | - | - | - | - | - | - | - | 1 | - | - |
| | Lipid composition H | - | - | - | - | - | - | - | - | 1 | - |
| | ceramide 2 (used alone) | - | - | - | - | - | - | - | - | - | 0.3 |
| Adhesiveness to skin (3days N/inch) | | 4.5 [Interfacial peeling] | 1.3 [Interfacial peeling] | 1.1 [Interfacial peeling] | 1.4 [Interfacial peeling] | 1.6 [Interfacial peeling] | 1.5 [Interfacial peeling] | 1.6 [Interfacial peeling] | 1.5 [Interfacial peeling] | 1.3 [Interfacial peeling] | 2.71 [Interfacial peeling] |
| Adhesiveness to Bakelite plate (N/inch) | | 5.5 [Interfacial peeling] | 4.4 [Interfacial peeling] | Not examined | Not examined | Not examined | Not examined | Not examined | Not examined | Not examined | 5.4 [Interfacial peeling] |
| TEWL recovery rate (%) (3days) | | 65 | 73 | 71 | 61 | 50 | 55 | 86 | 85 | 84 | 36 |
| Maltese cross image | | Found | Found | Found | Found | Found | Found | Not examined | Not examined | Not examined | Not examined |

The adhesive compositions of Examples 1 to 6 containing Lipid composition A and the adhesive compositions of Examples 7 to 9 containing Lipid composition F, G, or H had appropriate adhesiveness, and showed TEWL recovery rates higher than that of the adhesive composition of Comparative Example 1. In the patch including a dermatological adhesive disposed on a base sheet, achievement of an adhesive force to the skin according to the applications depend not only on the adhesiveness of the dermatological adhesive but also on the characteristics of the base sheet, such as flexibility. In this view, the adhesive compositions of Examples 2 to 9 do not necessarily have inferior adhesiveness to the adhesive composition of Comparative Example 1.

The results of the test suggest that the TEWL recovery rates of the adhesive compositions of Examples 1 to 9 higher than that of the adhesive composition of Comparative Example 1 are attributed to Lipid composition A, F, G, or H. These results lead to a conclusion that use of a lipid composition capable of forming a lamella structure rather than use of Ceramide 2 alone can enhance the TEWL recovery rate and the skin protective effect.

<Example 10>

Adhesive compositions mainly containing polyisobutylene as skeletal components (main components) of adhesives will be described.

Hydrogenated styrene-butadiene rubber (HSBR; made by JSR Corporation) (0.5 mass%) as a skeleton reinforcing component and liquid paraffin (Hicall K-350 made by KANEDA Co., Ltd.) as a plasticizer (0.2 mass%) were kneaded to yield a sufficiently homogeneous mixture. Polyisobutylene (Himol 6H made by JX Nippon Oil & Energy Corporation) (72.4 mass%) as a skeletal component and a vinyl acetate copolymer (hereinafter, abbreviated to EVA) (5.5 mass%) as a skeleton reinforcing component were then added, and were kneaded to give a homogeneous mixture. Silica (6.8 mass%) as a filler, carboxymethylcellulose sodium (CMC·Na) (13.0 mass%) as a hydrophilic polymer, citric acid (0.6 mass%) as a pH adjuster, and Lipid composition A (1 mass%) were mixed with stirring to prepare an adhesive composition of Example 10.

<Example 11>

An adhesive composition of Example 11 was prepared in a similar way to Example 10 except that the content of polyisobutylene was 58.7 mass% and a tackifier (13.7 mass%) was further added.

<Example 12>

An adhesive composition of Example 12 was prepared in a similar way to Example 10 except that the content of the hydrogenated styrene-butadiene rubber was 3.4 mass%, the content of the liquid paraffin 1.4 mass%, the content of the EVA 4.8 mass%, and the content of the polyisobutylene 69.0 mass%.

<Example 13>

An adhesive composition of Example 13 was prepared in a similar way to Example 10 except that the content of the polyisobutylene was 62.2 mass% and the content of the EVA was 15.7 mass.

<Example 14>

An adhesive composition of Example 14 was prepared in a similar way to Example 11 except that Lipid composition F (1 mass%) was compounded instead of Lipid composition A used in preparation of the adhesive composition of Example 11.

<Example 15>

An adhesive composition of Example 15 was prepared in a similar way to Example 11 except that Lipid composition G (1 mass%) was compounded instead of Lipid composition A used in preparation of the adhesive composition of Example 11.

<Example 16>

An adhesive composition of Example 16 was prepared in a similar way to Example 11 except that Lipid composition H (1 mass%) was compounded instead of Lipid composition A used in preparation of the adhesive composition of Example 11.

<Example 17>

An adhesive composition of Example 17 was prepared as follows: Without using hydrogenated styrene-butadiene rubber as a skeleton reinforcing component or liquid paraffin as a plasticizer, polyisobutylene (Himol 6H made by JX Nippon Oil & Energy Corporation) (65.6 mass%) as a skeletal component, EVA (6.1 mass%) as a skeleton reinforcing component, PE-modified silicone (6.8 mass%), silylated silica (6.9 mass%) as a filler, carboxymethylcellulose sodium (CMC·Na) (13.0 mass%) as a hydrophilic polymer, citric acid (0.6 mass%) as a pH adjuster, and Lipid composition A (1 mass%) were mixed with stirring. The PE-modified silicone was compounded to minimize the stickiness of the adhesive composition of Example 17.

(Evaluation of Examples 10 to 17)

The adhesive compositions prepared in Examples 10 to 17 were each extruded onto releasing paper at 75°C with a rolling mill into a thickness of 1.5 mm. The products were each applied onto a polyethylene film or a base sheet. Patches of Examples 10 to 17 were thereby prepared, in which the adhesives (adhesive layers) formed of the adhesive compositions of Examples 10 to 17 were disposed on the base sheets. Patches were evaluated after the releasing paper was removed.
The TEWL recovery rates of the patches prepared with the adhesive compositions of Examples 10 to 17 were determined in a similar way to Example 1.

(Adhesiveness to skin)

The patches prepared with the adhesive compositions of Examples 10 to 17 were cut into circular samples having a diameter of 30 mm. The samples were applied onto the bellies of subjects for six hours. The samples were evaluated whether these samples peeled off from the bellies or not and whether the adhesive was left or not. The sample was ranked as "A" if the sample did not peel off from the subject. The samples were evaluated according to the following three ranks:

A: The sample did not peel off within six hours, and no adhesive was left.
B: The sample did not peel off within six hours, but the adhesive was left.
C: The sample peeled off within six hours, and the adhesive was left.

(Presence of maltese cross image)

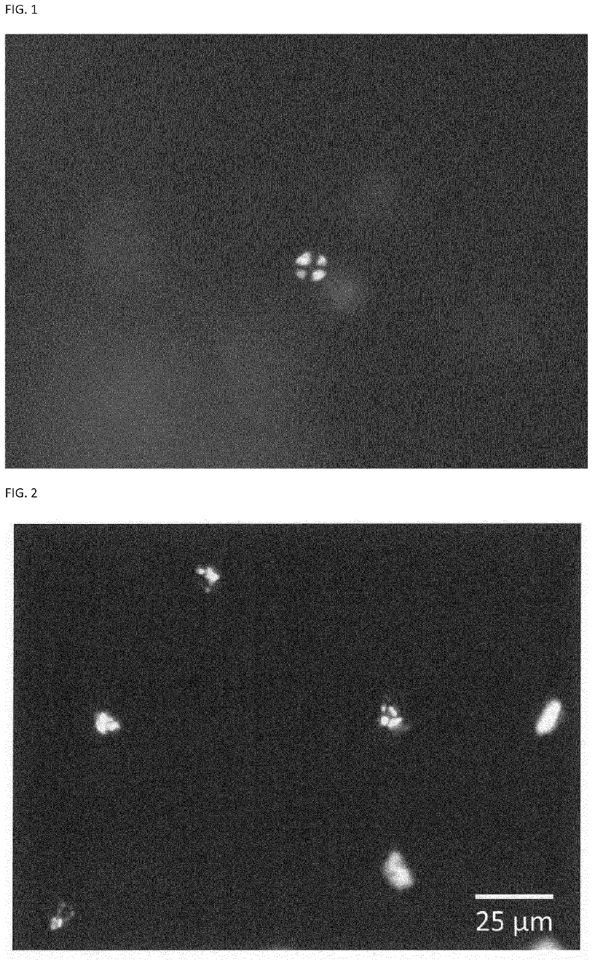

To confirm a lamellar structure in the adhesive layer formed of each adhesive composition, maltese cross images, which are inherent in the lamella structure, in the adhesive layers formed of adhesive compositions for observation were observed with a polarizing microscope.
The adhesive compositions for observation were prepared each Examples, in which, however, silica, citric acid, the tackifier, and CMC·Na were not included.
The compositions of the adhesive compositions of Examples 10 to 17 and the results in the measurement of the patches of Examples 10 to 17 are shown in Table 3. An image of the adhesive layer corresponding to Example 11 examined with a polarizing microscope is shown in Fig. 1.

[Table 3]

| Composition (mass%) | | Example 10 | Example 11 | Example 12 | Example 13 | Example 14 | Example 15 | Example 16 | Example 17 |
|---|---|---|---|---|---|---|---|---|---|
| | HSBR | 0.5 | 0.5 | 3.4 | 0.5 | 0.5 | 0.5 | 0.5 | - |
| | Liquid paraffin | 0.2 | 0.2 | 1.4 | 0.2 | 0.2 | 0.2 | 0.2 | - |
| | EVA | 5.5 | 5.5 | 4.8 | 15.7 | 5.5 | 5.5 | 5.5 | 6.1 |
| | Polyisobutylene | 72.4 | 58.7 | 69 | 62.2 | 58.7 | 58.7 | 58.7 | 65.6 |
| | Tackifier | - | 13.7 | - | - | 13.7 | 13.7 | 13.7 | - |
| | PE-modified silicone | - | - | - | - | - | - | - | 6.8 |
| | Silica | 6.8 | 6.8 | 6.8 | 6.8 | 6.8 | 6.8 | 6.8 | - |
| | Silylated silica | - | - | - | - | - | - | - | 6.9 |
| | CMC·Na | 13 | 13 | 13 | 13 | 13 | 13 | 13 | 13 |
| | Citric acid | 0.6 | 0.6 | 0.6 | 0.6 | 0.6 | 0.6 | 0.6 | 0.6 |
| | Lipid composition A | 1 | 1 | 1 | 1 | - | - | - | 1 |
| | Lipid composition F | - | - | - | - | 1 | - | - | - |
| | Lipid composition G | - | - | - | - | - | 1 | - | - |
| | Lipid composition H | - | - | - | - | - | - | 1 | - |
| | Ceramide 2 (used alone) | | | | - | - | - | - | - |
| Adhesiveness to skin (qualitative evaluation) | | A | A | A | A | A | A | A | A |
| TEWL recovery rate (%) (3days) | | 46.5 | 52.2 | 62.2 | 58.6 | 66.7 | 52 | 59.8 | 66.2 |
| Maltese cross image | | Found | Found | Found | Found | Not examined | Not examined | Not examined | Found |

The adhesive compositions of Example 10 to 16 containing polyisobutylene as a skeletal component, EVA as a skeleton reinforcing component, and Lipid composition A, F, G, or H had appropriate skin adhesiveness and showed high TEWL recovery rates. The results suggest that the adhesive compositions of Example 10 to 16 are useful as dermatological adhesives.

The adhesive composition of Example 17 containing polyisobutylene as a skeletal component, EVA as a skeleton reinforcing component, and Lipid composition A and not containing hydrogenated styrene-butadiene rubber as a skeleton reinforcing component and liquid paraffin as a plasticizer also had appropriate skin adhesiveness and a high TEWL recovery rate. The results suggest that the adhesive composition of Example 17 is useful as a dermatological adhesive.

<Example 18>

In Examples 10 to 17, a skeletal component polyisobutylene and a skeleton reinforcing component EVA were used as preferred examples.

In Example 18, polyisobutylene (41.0 mass%) as a skeletal component, karaya gum (20.0 mass%), pectin (10.0 mass%), gelatin (12.0 mass%), and guar gum (12.0 mass%) as hydrophilic polymers, silica (4.0 mass%) as a filler, and Lipid composition A (1.0 mass%) were placed in a pressurizing kneader, and were mixed under pressure to yield a sufficiently homogeneous mixture. An adhesive composition of Example 18 was thereby prepared.

<Example 19>

An adhesive composition of Example 19 was prepared in a similar way to Example 18 except that Lipid composition F (1.0 mass%) was compounded instead of Lipid composition A.

<Example 20>

An adhesive composition of Example 20 was prepared in a similar way to Example 18 except Lipid composition G (1.0 mass%) was compounded instead of Lipid composition A.

<Example 21>

An adhesive composition of Example 21 was prepared in a similar way to Example 18 except that Lipid composition H (1.0 mass%) was compounded instead of Lipid composition A.

(Evaluation of Examples 18 to 21)

The adhesiveness to the skin of the patches prepared with the adhesive compositions of Examples 18 to 21 were evaluated in a similar way to Examples 10 to 17. The TEWL recovery rates of the patches were determined in a similar way to Example 1. Furthermore, a maltese cross image was also examined in Example 18 with a polarizing microscope.

(Adhesiveness to skin)

The patches prepared with the adhesive compositions of Examples 18 to 21 were cut into circular samples having a diameter of 30 mm. The samples were applied onto the bellies of subjects for six hours. The samples were evaluated whether these samples peeled off from the bellies or not and whether the adhesive was left or not. The sample was ranked as "A" if the sample did not peel off from the subject. The samples were evaluated according to the following three ranks:

A: The sample did not peel off within six hours, and no adhesive was left.
B: The sample did not peel off within six hours, but the adhesive was left.
C: The sample peeled off within six hours, and the adhesive was left.

The compositions of the adhesive compositions of Examples 18 to 21 and the results in the measurement of the patches of Examples 18 to 21 are shown in Table 4.

[Table 4]

|  |  | Example 18 | Example 19 | Example 20 | Example 21 |
|---|---|---|---|---|---|
| Composition (mass%) | Polyisobutylene | 41 | 41 | 41 | 41 |
|  | Karaya gum | 20 | 20 | 20 | 20 |
|  | Pectin | 10 | 10 | 10 | 10 |
|  | Gelatin | 12 | 12 | 12 | 12 |
|  | Guar gum | 12 | 12 | 12 | 12 |
|  | Silica | 4 | 4 | 4 | 4 |
|  | Lipid composition A | 1 | - | - | - |
|  | Lipid composition F |  | 1 | - | - |
|  | Lipid composition G | - | - | 1 | - |
|  | Lipid composition H | - | - | - | 1 |
| Adhesiveness to skin (qualitative evaluation) |  | A | A | A | A |
| TEWL recovery rate (%) (3days) |  | 54 | 66 | 62 | 60 |
| Maltese cross image |  | Found | Not examined | Not examined | Not examined |

The adhesive compositions of Examples 18 to 21 containing polyisobutylene as a skeletal component, karaya gum, pectin, gelatin, and guar gum as hydrophilic polymers, and Lipid composition A, F, G, or H had appropriate skin adhesiveness and showed high TEWL recovery rates. The results suggest that the adhesive compositions of Examples 18 to 21 are useful as dermatological adhesives.

<Example 22>

An ultraviolet light curable resin (trade name "acResin A260 UV" made by BASF SE, a polymer including a structural unit derived from butyl acrylate) (75.5 mass%) as a radiation curable resin, PO-modified trimethylolpropane triacrylate (TMP-3P made by Dai-ichi Kogyo Seiyaku Co., Ltd., viscosity at 25°C: 60 mPa·s) (2.5 mass%) as a reactive diluent, a functional group-free acrylic polymer (ARUFON (registered trademark) made by TOAGOSEI CO., LTD.) UP-1000, Mw: 3000, viscosity at 25°C: 1000 mPa·s, Tg: -77°C, solid content: 98% or more) (10 mass%), CMC·Na (10 mass%), silica (1 mass%), and Lipid composition A (1 mass%) were mixed with stirring to prepare an adhesive composition of Example 22.
<Comparative Example 2>
An adhesive composition of Comparative Example 2 was prepared in a similar way to Example 22 except that the content of the ultraviolet light curable resin was 76.4 mass% and Ceramide 2 (0.1 mass%) was compounded instead of Lipid composition A.

<Example 23>

An adhesive composition of Example 23 was prepared in a similar way to Example 22 except that the content of the ultraviolet light curable resin was 75 mass% and silicone oil ("SH3772M" made by Dow Corning Toray Co., Ltd.) (0.5 mass%) was further compounded.

<Comparative Example 3>

An adhesive composition of Comparative Example 3 was prepared in a similar way to Example 22 except that the content of the ultraviolet light curable resin was 75.9 mass%, silicone oil ("SH3772M" made by Dow Corning Toray Co., Ltd.) (0.5 mass%) was further compounded, and Ceramide 2 (0.1 mass%) was compounded instead of Lipid composition A.

<Example 24>

An adhesive composition of Example 24 was prepared in a similar way to Example 23 except that Lipid composition A was replaced with Lipid composition B.

<Example 25>

An adhesive composition of Example 25 was prepared in a similar way to Example 23 except that the content of the ultraviolet light curable resin was 75.1 mass% and Lipid composition A was replaced with Lipid composition C (0.9 mass%).

<Example 26>

The ultraviolet light curable resin used in Example 22 (98.5 mass%), the silicone oil used in Example 23 (0.5 mass%), and Lipid composition A (1 mass%) were compounded to prepare an adhesive composition of Example 26.

<Example 27>

The ultraviolet light curable resin used in Example 22 (70 mass%), the reactive diluent used in Example 22 (5 mass%), the functional group-free acrylic polymer used in Example 22 (10 mass%), the silica used in Example 22 (1 mass%), CMC·Na used in Example 22 (13 mass%), and Lipid composition D (1 mass%) were compounded to prepare an adhesive composition of Example 27.

<Example 28>

The ultraviolet light curable resin used in Example 22 (85 mass%), the reactive diluent used in Example 22 (2.5 mass%), the functional group-free acrylic polymer used in Example 22 (2.0 mass%), the silica used in Example 22 (1 mass%), CMC·Na used in Example 22 (8.5 mass%), and Lipid composition A (1 mass%) were compounded to prepare an adhesive composition of Example 28.

<Example 29>

The ultraviolet light curable resin used in Example 22 (65 mass%), the reactive diluent used in Example 22 (2.5 mass%), the functional group-free acrylic polymer used in Example 22 (12.5 mass%), the silica used in Example 22 (1 mass%), CMC·Na used in Example 22 (18 mass%), and Lipid composition A (1 mass%) were compounded to prepare an adhesive composition of Example 29.

<Example 30>

An adhesive composition of Example 30 was prepared in a similar way to Example 22 except that Lipid composition A was replaced with Lipid composition E (1 mass%).

<Example 31>

An adhesive composition of Example 31 was prepared in a similar way to Example 22 except that Lipid composition A was replaced with Lipid composition F (1 mass%).

<Example 32>

An adhesive composition of Example 32 was prepared in a similar way to Example 22 except that Lipid composition A was replaced with Lipid composition G (1 mass%).

<Example 33>

An adhesive composition of Example 30 was prepared in a similar way to Example 22 except that Lipid composition A was replaced with Lipid composition H (1 mass%).

<Example 34>

An adhesive composition of Example 34 was prepared in a similar way to Example 22 except that Lipid composition A was replaced with Lipid composition I (1 mass%).

(Evaluation of Examples 22 to 34 and Comparative Examples 2 and 3)

The adhesive compositions prepared in Examples 22 to 34 and Comparative Examples 2 and 3 were applied onto PET releasing films (thickness: 25 $\mu$m) at 120°C with a coater into a thickness of 100 $\mu$m. The adhesive compositions applied onto the PET releasing films were disposed on polyolefin non-woven fabric base sheets. The adhesive compositions were then laminated to the PET releasing films at 80°C and 0.4 MPa, and the PET releasing films were removed. The adhesive compositions were irradiated with ultraviolet light from an ultraviolet light irradiating apparatus such that the accumulated dose was 200 mJ/cm$^2$. Patches having dermatological adhesives formed on the polyolefin non-woven fabrics were thereby prepared. The patches were evaluated after aged at 60°C for five days.

In the patches prepared with the adhesive compositions of Examples 22 to 34 and Comparative Examples 2 and 3, the adhesiveness and the TEWL recovery rate were determined in a similar way to Example 1.

(Presence of maltese cross image)

To confirm whether a lamellar structure in the adhesive layer formed of each adhesive composition is found, maltese cross images in the adhesive layers formed of adhesive compositions for observation were examined with a polarizing microscope.

The adhesive compositions for observation in Examples 22 to 29 were prepared with ultraviolet light curable resins containing the lipid composition used in Examples 22 to 29. The adhesive compositions for observation in Comparative Examples 2 and 3 were prepared with ultraviolet light curable resins containing Ceramide 2 used in Comparative Examples 2 and 3.

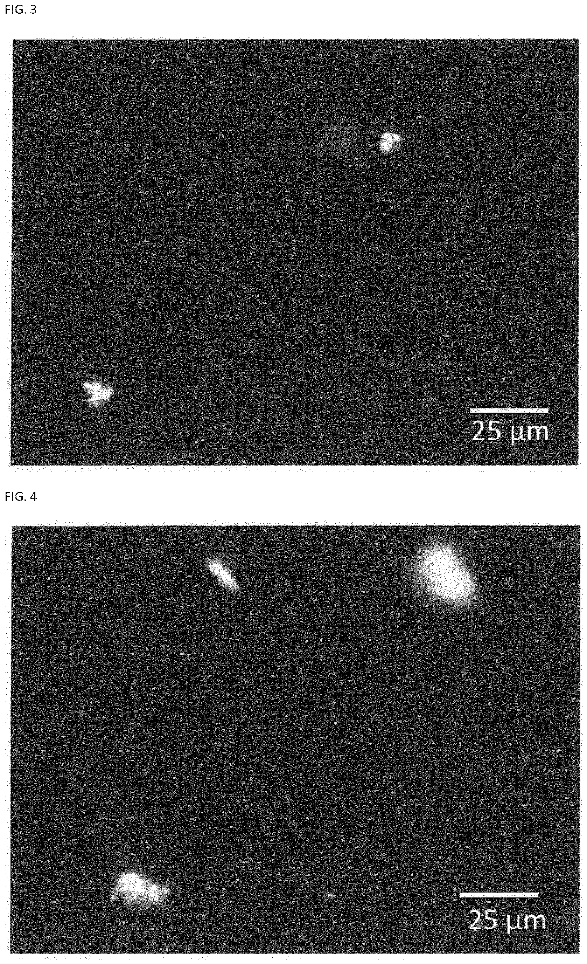

The compositions of the adhesive compositions of Examples 22 to 34 and Comparative Examples 2 and 3 and the results in the measurement of the patches of Examples 22 to 34 and Comparative Examples 2 and 3 are shown in Tables 5 to 7. Images obtained through examination of the adhesive layers of Examples 22, 24, and 25 with a polarizing microscope are shown in Figs. 2 to 4.

[Table 5]

| | | Example 22 | Comparative Example 2 | Example 23 | Comparative Example 3 |
|---|---|---|---|---|---|
| Composition (mass%) | Ultraviolet light curable resin | 75.5 | 76.4 | 75 | 75.9 |
| | Silicone oil | - | - | 0.5 | 0.5 |
| | Reactive diluent | 2.5 | 2.5 | 2.5 | 2.5 |
| | Functional group-free acrylic polymer | 10 | 10 | 10 | 10 |
| | CMC·Na | 10 | 10 | 10 | 10 |
| | Silica | 1 | 1 | 1 | 1 |
| | Lipid composition A | 1 | - | 1 | - |
| | Ceramide 2 (used alone) | - | 0.1 | - | 0.1 |
| Adhesiveness to skin (N/25mm) | | 2.8 [Interfacial peeling] | 3.8 [Interfacial peeling] | 2.1 [Interfacial peeling] | 2.5 [Interfacial peeling] |
| Adhesiveness to Bakelite plate (N/25mm) | | 7.7 [Interfacial peeling] | 8.9 [Interfacial peeling] | 6.9 [Interfacial peeling] | 7.9 [Interfacial peeling] |
| TEWL recovery rate (%) (3days) | | 43.6 | 18.1 | 24.7 | -49.5 |
| Maltese cross image | | Found | Not found | Found | Not found |

[Table 6]

| Composition (mass%) | | Example 24 | Example 25 | Example 26 | Example 27 |
|---|---|---|---|---|---|
| Composition (mass%) | Ultraviolet light curable resin | 75 | 75.1 | 98.5 | 70 |
| | Silicone oil | 0.5 | 0.5 | 0.5 | - |
| | Reactive diluent | 2.5 | 2.5 | - | 5 |
| | Functional group-free acrylic polymer | 10 | 10 | - | 10 |
| | CMC·Na | 10 | 10 | - | 13 |
| | Silica | 1 | 1 | - | 1 |
| | Lipid composition A | - | - | 1 | |
| | Lipid composition B | 1 | - | - | - |
| | Lipid composition C | - | 0.9 | - | - |
| | Lipid composition D | - | - | - | 1 |
| Adhesiveness to skin (N/25mm) | | 2.1 [Interfacial peeling] | 2.2 [Interfacial peeling] | 0.1 [Interfacial peeling] | 1.2 [Interfacial peeling] |
| Adhesiveness to Bakelite plate (N/25mm) | | 7.2 [Interfacial peeling] | 4.9 [Interfacial peeling] | 2.3 [Interfacial peeling] | 6.1 [Interfacial peeling] |
| TEWL recovery rate (%) (3days) | | -20.8 | 14.8 | 58.7 | 81.5 |
| Maltese cross image | | Found | Found | Found | Found |

28

[Table 7]

| | | Example 28 | Example 29 | Example 30 | Example 31 | Example 32 | Example 33 | Example 34 |
|---|---|---|---|---|---|---|---|---|
| Composition (mass%) | Ultraviolet light curable resin | 85 | 65 | 75.5 | 75.5 | 75.5 | 75.5 | 75.5 |
| | Silicone oil | - | - | - | - | - | - | - |
| | Reactive diluent | 2.5 | 2.5 | 2.5 | 2.5 | 2.5 | 2.5 | 2.5 |
| | Functional group-free acrylic polymer | 2 | 12.5 | 10 | 10 | 10 | 10 | 10 |
| | CMC·Na | 8.5 | 18 | 10 | 10 | 10 | 10 | 10 |
| | Silica | 1 | 1 | 1 | 1 | 1 | 1 | 1 |
| | Lipid composition A | 1 | 1 | - | - | - | - | - |
| | Lipid composition E | - | - | 1 | - | - | - | - |
| | Lipid composition F | - | - | - | 1 | - | - | - |
| | Lipid composition G | - | - | - | - | 1 | - | - |
| | Lipid composition H | - | - | - | - | - | 1 | - |
| | Lipid composition I | - | - | - | - | - | - | 1 |
| Adhesiveness to skin (N/25mm) | | 2.1 [Interfacial peeling] | 3.1 [Interfacial peeling] | 2.9 [Interfacial peeling] | 2.7 [Interfacial peeling] | 3.7 [Interfacial peeling] | 2.3 [Interfacial peeling] | 3.7 [Interfacial peeling] |
| Adhesiveness to Bakelite plate (N/25mm) | | 9.8 [Interfacial peeling] | 11.3 [Interfacial peeling] | 15.3 [Interfacial peeling] | 19.8 [Interfacial peeling] | 12.9 [Interfacial peeling] | 7.9 [Interfacial peeling] | 10 [Interfacial peeling] |
| TEWL recovery rate (%) (3days) | | 24.7 | 63.4 | 26.1 | 55.9 | 54.2 | 72.9 | 74.9 |
| Maltese cross image | | Found | Found | Not examined | Not examined | Not examined | Not examined | Not examined |

The adhesive layers for observation formed of the adhesive compositions for observation corresponding to the adhesive compositions of Examples 22 to 34 and Comparative Examples 2 and 3 were examined with a polarizing microscope. Maltese cross images were found in the adhesive layers formed of the adhesive compositions for observation corresponding to the adhesive compositions of Examples 22 to 34 (see Figs. 2 to 4). These results suggest that the Lipid compositions A to I formed lamella structures.

Accordingly, the adhesive composition according to the present embodiment provides a dermatological adhesive composition comprising a resin component and an appropriate lipid composition such that maltese cross images can be found through examination with a polarizing microscope, or a dermatological patch containing the dermatological adhesive composition.

Comparison between Example 22 and Comparative Example 2, in which the TEWL recovery rates were measured on Day 3 after application of the patches, and comparison between Example 23 and Comparative Example 3, in which the TEWL recovery rates were measured on Day 1 after application of the patches suggested that the TEWL recovery rate was enhanced more significantly in use of a lipid composition capable of forming a lamella structure than in use of Ceramide 2 alone.

These results and the results in Examples 24 and 25 suggested that the TEWL recovery rate was enhanced by a lipid composition capable of forming a lamella structure. Although the TEWL recovery rate of Example 24 is a negative value, it still suggested that the TEWL recovery rate was enhanced more significantly than in Comparative Example 3 using Ceramide 2 alone and showing a TEWL recovery rate measured after the same period had passed as in Example 24. These results lead to a conclusion that an adhesive composition containing a lipid composition capable of forming a lamella structure can significantly enhance the skin protective effect.

<Example 35>

Examples of an adhesive composition containing an emulsion adhesive as a resin component and Ceramide 2 as a lamellar structure will now be described.

Lipid composition A (0.45 g) was suspended in 5 g of water, and was heated until being dissolved to prepare a lipid composition solution. The lipid composition solution was added to an emulsion adhesive (100 g), and was mixed with stirring to prepare an adhesive composition of Example 35.

<Example 36>

An adhesive composition of Example 36 was prepared in a similar way to Example 35 except that the amount of Lipid composition A was 0.045 g.

(Evaluation of Examples 35 and 36)

The adhesive compositions prepared in Examples 35 and 36 were each applied to silicone releasing paper, and the coatings were dried at 100°C for two minutes. The products were applied to urethane films to prepare patches having the dermatological adhesives of Examples 35 and 36. The adhesive layer of each patch had a thickness of about 25 μm. These patches were aged at 40°C for 24 hours. After the releasing paper was removed, the patches were evaluated as follows.

The adhesiveness to the skin, the adhesiveness to the Bakelite plate, and the TEWL recovery rate were determined in a similar way to Examples 1 to 9. Maltese cross images were also observed.

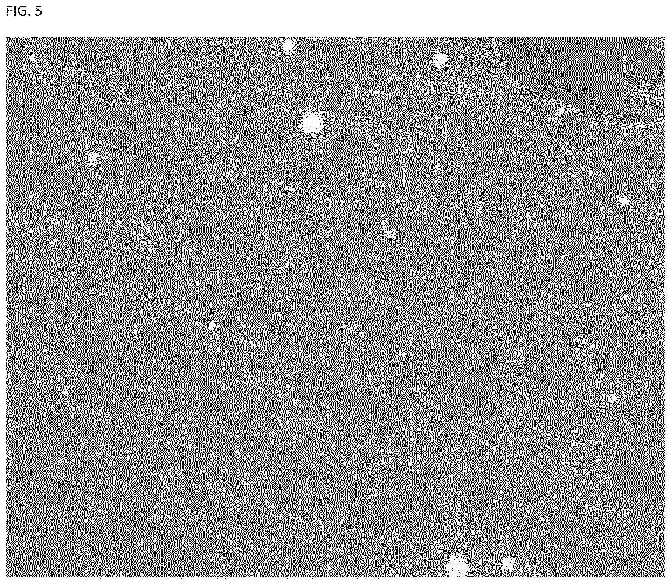

The compositions of the adhesive compositions of Examples 35 and 36 and the results in the measurement of the patches of Examples 35 and 36 are shown in Table 8. In the composition, the solid content (mass%) of the emulsion adhesive is shown. An image obtained through examination of the adhesive layer corresponding to Example 35 with a polarizing microscope is shown in Fig. 5.

[Table 8]

|  |  | Example 35 | Example 36 |
|---|---|---|---|
| Composition (mass%) | Emulsion adhesive (solid content) | 99.56 | 99.96 |
|  | Lipid composition A | 0.44 | 0.04 |
| Adhesiveness to skin (N/25mm) |  | 1.61 | 1.53 |
| Adhesiveness to Bakelite plate (N/25mm) |  | 11.41 | 10.38 |
| TEWL recovery rate (%) (3days) |  | 67 | 63 |

(continued)

|  | Example 35 | Example 36 |
|---|---|---|
| Maltese cross image | Found | Found |

An adhesive composition containing an emulsion adhesive and ceramide as a lamellar structure also had appropriate adhesiveness and an appropriate TEWL recovery rate.

<Example 37>

Examples of adhesive compositions containing silicone adhesives as resin components and Ceramide 2 as a lamellar structure will now be described.
A mixture (35.6 mass%) of a two-part silicone composition containing an alkenyl group-containing polyorganopolysiloxane and organohydrogenpolysiloxane as main components (made by Dow Corning Corp., trade name "Q7-9700 SS Adhesive"), an adhesive silicone component (KR-3701 made by Shin-Etsu Chemical Co., Ltd.) (60.7 mass%), a platinum catalyst (3.0 mass%), and Lipid composition A (0.7%) were mixed with stirring to prepare an adhesive composition of Example 37.

<Example 38>

An adhesive composition of Example 38 was prepared in a similar way to Example 37 except that the content of the mixture of the silicone composition was 35.8 mass%, the content of the adhesive silicone component was 61.1%, and the content of Lipid composition A was 0.1 mass%.

(Evaluation of Examples 37 and 38)

The adhesive compositions prepared in Examples 37 and 38 were applied onto silicone releasing paper, and were heated at 100°C for three minutes to be cured. The products were applied to urethane films to prepare patches having dermatological adhesives. The adhesive layer of each patch had a thickness of about 45 $\mu$m. The patches were aged at 40°C for 24 hours. After the releasing paper was removed, the patches were evaluated as follows.
The adhesiveness to the skin, the adhesiveness to the Bakelite plate, and the TEWL recovery rate were determined in a similar way to Examples 1 to 9. Maltese cross images were also observed.
The compositions of the adhesive compositions of Examples 37 and 38 and the results in the measurement of the patches of Examples 37 and 38 are shown in Table 9.

[Table 9]

|  |  | Example 37 | Example 38 |
|---|---|---|---|
| Composition (mass%) | Silicone composition | 35.6 | 35.8 |
|  | Adhesive silicone component | 60.7 | 61.1 |
|  | Platinum catalyst | 3.0 | 3.0 |
|  | Lipid composition A | 0.7 | 0.1 |
| Adhesiveness to skin (N/25mm) | | 2 | 1.9 |
| Adhesiveness to Bakelite plate (N/25mm) | | 2.7 | 1.9 |
| TEWL recovery rate (%) (3days) | | 81 | 76 |
| Maltese cross image | | Found | Found |

The adhesive compositions containing Lipid composition A according to the present embodiment, the silicone compositions, and the adhesive silicone components also had appropriate adhesiveness to the skin and high TEWL recovery rates. These results suggested that the adhesive compositions are useful as dermatological adhesives.

**Claims**

1. A dermatological adhesive composition comprising:

    a resin component, and
    a lamellar structure comprising a lipid composition.

2. The dermatological adhesive composition according to claim 1,

    wherein the lamellar structure comprises a lipid composition comprising a sphingolipid and/or a sterol.

3. The dermatological adhesive composition according to claim 1 or 2,

    wherein the lamellar structure comprises a lipid composition comprising at least one selected from the group consisting of sphingolipids, sterols, and surfactants; and higher alcohol.

4. The dermatological adhesive composition according to any one of claims 1 to 3,

    wherein the lamellar structure comprises a lipid composition comprising a glycerol fatty acid ester, and an acylated lactic acid and/or a salt thereof.

5. The dermatological adhesive composition according to any one of claims 1 to 3,

    wherein the lamellar structure comprises a lipid composition comprising polyoxyalkylene alkyl ether or polyoxyalkylene glycol fatty acid ester as a surfactant.

6. The dermatological adhesive composition according to any one of claims 1 to 5,

    wherein the lamellar structure comprises the lipid composition comprising at least one selected from the group consisting of the sphingolipids, the sterols, and the surfactants in a total amount of 5 to 80 mass%, and the higher alcohol in a total amount of 10 to 50 mass%.

7. The dermatological adhesive composition according to any one of claims 1 to 5,

    wherein the lamellar structure comprises of the lipid composition comprising at least one selected from the group consisting of the sphingolipid, the sterol, and the glycerol fatty acid ester in a total amount of 5 to 80 mass%, and the higher alcohol in a total amount of 10 to 50 mass%.

8. The dermatological adhesive composition according to any one of claims 1 to 7,

    wherein the resin component comprises an acrylic resin serving as a resin component for forming a hot-melt adhesive.

9. The dermatological adhesive composition according to any one of claims 1 to 7,

    wherein the resin component comprises a styrene-based thermoplastic elastomer serving as a resin component for forming a hot-melt adhesive.

10. The dermatological adhesive composition according to any one of claims 1 to 9, further comprising a hydrophilic polymer.

11. A dermatological adhesive composition comprising:

    a resin component, and
    a lipid composition comprising at least one selected from the group consisting of sphingolipids and sterols, and at least one selected from the group consisting of higher alcohol and surfactants.

12. The dermatological adhesive composition according to claim 11,

wherein the surfactant comprises at least one selected from the group consisting of glycerol fatty acid esters, acylated lactic acids and/or salts thereof, polyoxyalkylene alkyl ethers, and polyoxyalkylene glycol fatty acid esters.

13. A dermatological patch comprising:

a base sheet, and
an adhesive layer formed of the dermatological adhesive composition according to any one of claims 1 to 12, and disposed on the base sheet.

FIG. 1

FIG. 2

25 μm

FIG. 3

25 μm

FIG. 4

25 μm

FIG. 5

## INTERNATIONAL SEARCH REPORT

| International application No. |
| --- |
| PCT/JP2015/064544 |

| A. CLASSIFICATION OF SUBJECT MATTER |
| --- |
| *A61K9/70*(2006.01)i, *A61K47/10*(2006.01)i, *A61K47/14*(2006.01)i, *A61K47/16* (2006.01)i, *A61K47/28*(2006.01)i, *A61K47/32*(2006.01)i, *A61L15/58*(2006.01)i, *A61P17/16*(2006.01)i |
| According to International Patent Classification (IPC) or to both national classification and IPC |

| B. FIELDS SEARCHED |
| --- |
| Minimum documentation searched (classification system followed by classification symbols) A61K9/70, A61K47/10, A61K47/14, A61K47/16, A61K47/28, A61K47/32, A61L15/58, A61P17/16 |

| Documentation searched other than minimum documentation to the extent that such documents are included in the fields searched |
| --- |
| Jitsuyo Shinan Koho 1922-1996 Jitsuyo Shinan Toroku Koho 1996-2015 Kokai Jitsuyo Shinan Koho 1971-2015 Toroku Jitsuyo Shinan Koho 1994-2015 |

| Electronic data base consulted during the international search (name of data base and, where practicable, search terms used) JSTPlus/JMEDPlus/JST7580(JDreamIII) |
| --- |

| C. DOCUMENTS CONSIDERED TO BE RELEVANT |
| --- |

| Category* | Citation of document, with indication, where appropriate, of the relevant passages | Relevant to claim No. |
| --- | --- | --- |
| X<br>A | WO 2012/63947 A1 (Nissan Chemical Industries, Ltd.),<br>18 May 2012 (18.05.2012),<br>claims; paragraphs [0026], [0035] to [0038], [0070]; examples; fig. 9<br>& US 2013/0296761 A1 & EP 2638921 A1 | 1,2,4,5,<br>8-10,13<br>3,6,7,11-12 |
| A | JP 2006-263042 A (Alcare Co., Ltd.),<br>05 October 2006 (05.10.2006),<br>entire text<br>(Family: none) | 1-13 |
| A | JP 11-505843 A (Alza Corp.),<br>25 May 1999 (25.05.1999),<br>entire text<br>& US 5882676 A & WO 1996/037231 A1<br>& EP 828516 A | 1-13 |

| ☐ Further documents are listed in the continuation of Box C. | ☐ See patent family annex. |
| --- | --- |

| * Special categories of cited documents: | "T" later document published after the international filing date or priority |
| --- | --- |
| "A" document defining the general state of the art which is not considered to be of particular relevance | date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 01 July 2015 (01.07.15) | Date of mailing of the international search report 14 July 2015 (14.07.15) |
| --- | --- |
| Name and mailing address of the ISA/ Japan Patent Office 3-4-3,Kasumigaseki,Chiyoda-ku, Tokyo 100-8915,Japan | Authorized officer<br><br>Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006263042 A

- JP 2009087877 W

**Non-patent literature cited in the description**

- Handobukku -Keshohin/Seizai Zairyo. Nikko Chemicals Co., Ltd, 01 February 1977